# EUROPEAN PATENT APPLICATION

(11) **EP 0 794 185 A1**
(43) Date of publication of application: **10.09.1997**
(21) Application number: 95937193.1
(22) Date of filing: 27.11.1995
(51) Int. Cl.: C07D 519/00, A61K 31/425

(54) **BICYCLIC THIAZOLE COMPOUND**

(30) Priority: 25.11.1994 JP 291054/94
(71) Applicant: MEIJI SEIKA KABUSHIKI KAISHA, Tokyo 104 (JP)
(72) Inventor: FUKUDA, Yoshimasa Pharmaceutical Research Center, Kouhoku-ku, Yokohama-shi Kanagawa 222 (JP); HASEGAWA, Toshifumi Pharmaceutical Research Center, Kouhoku-ku, Yokohama-shi Kanagawa 222 (JP); NAKATANI, Yuuko Pharmaceutical Research Center, Kouhoku-ku, Yokohama-shi Kanagawa 222 (JP); MURASE, Kenshi Pharmaceutical Research Center, Kouhoku-ku, Yokohama-shi Kanagawa 222 (JP); FUJI, Kazuyuki Pharmaceutical Research Center, Kouhoku-ku, Yokohama-shi Kanagawa 222 (JP)
(74) Representative: Dowden, Marina
(86) International application number: JP9502412
(87) International publication number: WO9616968

(57) **Abstract**

A compound, represented by the following formula (I), having psychotropic effect is disclosed. A compound represented by the following formula (I) or a pharmaceutically acceptable salt thereof: 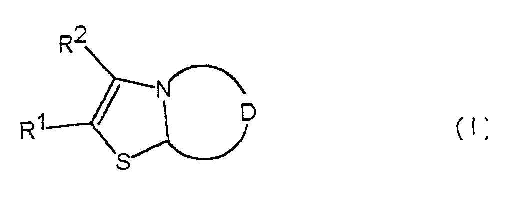 wherein
any one of R¹ and R² represents group W-(CH₂)ₘ- wherein m is an integer of 1 to 4 with the other representing H or optionally substituted lower alkyl,
group W is a group represented by the following formula (i): 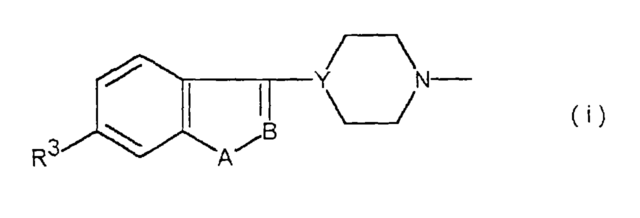 wherein
R³ represents H or a halogen atom,
A represents O or S,
B represents N or CH, and
Y represents CH or N; and
ring structure -D- represents any one of the following formulae (a) to (d): 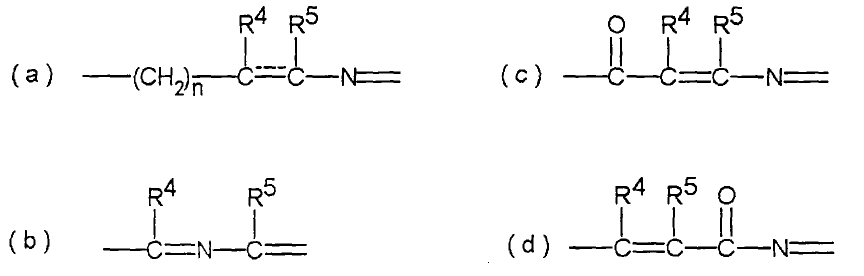 wherein
n is an integer of 0 to 2,
R⁴ and R⁵ each independently represent H, lower alkyl, a hydroxyl group, alkoxy, alkoxycarbonyl, or aminocarbonyl, and
a solid line accompanied by a dotted line represents a single bond or a double bond.

## Description

### [BACKGROUND OF THE INVENTION]

### Field of the Invention

The present invention relates to psychotropic agents comprising as an active ingredient a compound having a psychotropic activity or a pharmaceutically acceptable salt thereof.

### Background Art

Compounds, which are bicyclic thiazole derivatives and bonded to an amino group or its derivative through an alkylene group, are described in Chem. Chron., 9, 239 (1980). The amino group portion of these compounds is morpholine. Other compounds of this type are described in EP-2400 and J. Chem. Soc. Perkin Trans., 10, 2221 (1984).

### [SUMMARY OF THE INVENTION]

The present inventors have now found that certain bicyclic thiazole compounds have strong psychotropic activity. The present invention has been made based on such finding.

Accordingly, an object of the present invention is to provide a compound having psychotropic activity.

Another object of the present invention is to provide a psychotropic agent comprising the above derivative.

According to the present invention, there is provided a compound represented by the following formula (I) or a pharmaceutically acceptable salt thereof: 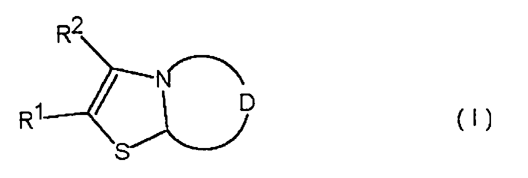 wherein
any one of R¹ and R² represents group W*-*(CH₂)ₘ- wherein m is an integer of 1 to 4 with the other representing H or optionally substituted lower alkyl, and
group W is a group represented by the following formula (i): 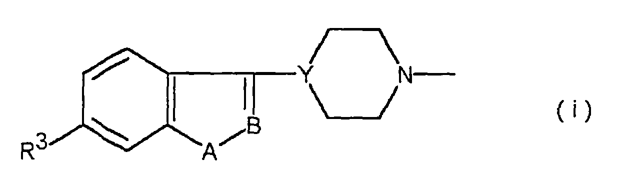 wherein
R³ represents H or a halogen atom,
A represents O or S,
B represents N or CH, and
Y represents CH or N; and
ring structure -D- represents any one of the following formulae (a) to (d): 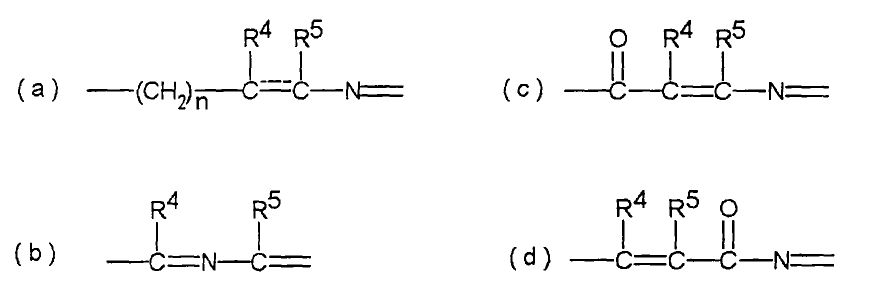 wherein
n is an integer of 0 to 2,
R⁴ and R⁵ each independently represent H, lower alkyl, a hydroxyl group, alkoxy, alkoxycarbonyl, or aminocarbonyl, and
a solid line accompanied by a dotted line represents a single bond or a double bond.

The compound represented by the formula (I) has strong psychotropic activity. Therefore, the present invention can provide psychotropic agents, which can effectively act on mentation, especially antischizophrenia agents, antianxiety agents, and antidepressants.

### [DETAILED DESCRIPTION OF THE INVENTION]

### Compound represented by the formula (I)

The term "lower alkyl" as a group or a portion of a group used herein means straight-chain or branched alkyl having 1 to 6 carbon atoms, preferably 1 to 4 carbon atoms. The term "acyloxy" used herein means lower alkylcarbonyloxy or arylcarbonyloxy. The term "aryl" used herein means phenyl or naphthyl. The term "halogen atom" means a fluorine, chlorine, bromine, or iodine atom.

In the formula (I), at least one hydrogen atom in the lower alkyl represented by any one of R¹ and R² may be substituted, and preferred examples of the substituent include a halogen atom, a hydroxyl group, acyloxy (for example, C₁₋₆ alkylcarbonyloxy, preferably C₁₋₄ alkylcarbonyloxy, benzoyloxy, or naphthoyloxy), alkoxy (for example, C_{1*-*6} alkoxy, preferably C_{1*-*4} alkoxy, or cyclic alkyloxy, preferably C_{4*-*7} cycloalkyloxy), lower alkylsulfonyloxy (for example, methanesulfonyloxy), and aryloxysulfonyloxy [for example, phenyloxysulfonyl optionally substituted by lower alkyl (for example, p-toluenesulfonyloxy), or naphthyloxysulfonyl optionally substituted by lower alkyl]. The lower alkyl represented by any one of R¹ and R² preferably represents methyl.

In the group represented by the formula (i) which is in turn represented by W, R³ preferably represents a halogen atom with a fluorine atom being more preferred.

Preferred groups represented by the formula (i) include:
groups wherein A represents an oxygen atom, B represents a nitrogen atom and Y represents CH, particularly groups wherein R³ represents a fluorine atom;
groups wherein A represents a sulfur atom, B represents a nitrogen atom and Y represents CH or a nitrogen atom, particularly groups wherein Y represents CH and R³ represents a hydrogen or fluorine atom;
groups wherein A represents an oxygen or sulfur atom,
B represents CH and Y represents CH, particularly groups wherein A represents an oxygen atom and R³ represents a fluorine atom.

The ring structure -D- in the formula (I) represents a structure represented by any one of the formulae (a) to (d). In these groups, R⁴ and R⁵ each independently represent a hydrogen atom, lower alkyl, a hydroxyl group, lower alkoxy, lower alkoxycarbonyl, or aminocarbonyl. R⁴ and R⁵ preferably represent a hydrogen atom, C_{1*-*6} alkyl (more preferably, C₁₋₄ alkyl), C_{1*-*6} alkoxycarbonyl (more preferably, C₁₋₄ alkyl) with any one of R⁴ and R⁵ being particularly preferably a hydrogen atom.

Specific examples of preferred compounds according to the present invention include:
3,7-dimethyl-2-[2-[4-(6-fluoro-1,2-benzisoxazol-3-yl)-1-piperidinyl]ethyl]-5H-thiazolo[3,2-a]pyrimidin-5-one,
3,7-dimethyl-2-[2-[4-(6-fluoro-1-benzothiophen-3-yl)-1-piperidinyl]ethyl]-5H-thiazolo[3,2-a]pyrimidin-5-one,
3,6-dimethyl-2-[2-[4-(6-fluoro-1,2-benzisoxazol-3-yl)-1-piperidinyl]ethyl]imidazo[2,1*-*b]thiazole,
3,6-dimethyl-2-[2-[4-(6-fluoro-1-benzothiophen-3-yl)-1-piperidinyl]ethyl]imidazo[2,1-b]thiazole,
3,5-dimethyl-2-[2-[4-(6-fluoro-1,2-benzisoxazol-3-yl)-1-piperidinyl]ethyl]-7H-thiazolo[3,2-a]pyrimidin-7-one,
3-[2-[4-(6-fluoro-1,2-benzisoxazol-3-yl)-1-piperidinyl]ethyl]-5H-7-methylthiazolo[3,2-a]pyrimidin-5-one,
6-ethoxycarbonyl-2-[2-[4-(6-fluoro-1,2-benzisoxazol-3-yl)-1-piperidinyl]ethyl]-5H-3-methylthiazolo[3,2-a]pyrimidin-5-one,
6-ethoxycarbonyl-2-[2-[4-(1,2-benzoisothiazol-3-yl)-1-piperazinyl]ethyl]-5H-3-methylthiazolo[3,2-a]pyrimidin-5-one,
3-[2-[4-(6-fluoro-1,2-benzisoxazol-3-yl)-1-piperidinyl]ethyl]-6-methylimidazo[2,1*-*b]thiazole,
3-[2-[4-(6-fluoro-1,2-benzisoxazol-3-yl)-1-piperidinyl]ethyl]-2-methylimidazo[2,1-b]thiazole,
3-[2-[4-(6-fluoro-1,2-benzisoxazol-3-yl)-1-piperidinyl]ethyl]-2,6-dimethylimidazo[2,1-b]thiazole,
3-[2-[4-(6-fluorobenzo[b]furan-3-yl)-1-piperidinyl]ethyl]-6-isopropylimidazo[2,1-b]thiazole,
3-[2-[4-(6-fluoro-1,2-benzisoxazol-3-yl)-1-piperidinyl]ethyl]-6-isopropyl-2-methylimidazo[2,1-b]thiazole,
3-[2-[4-(6-fluorobenzo[b]furan-3-yl)-1-piperidinyl]ethyl]-6-isopropyl-2-methylimidazo[2,1-b]thiazole,
3-[2-[4-(1,2-benzoisothiazol-3-yl)-1-piperazinyl]ethyl]-6-isopropyl-2-methylimidazo[2,1-b]thiazole,
3-[2-[4-(1,2-benzoisothiazol-3-yl)-1-piperazinyl]ethyl]-5H-7-methylthiazolo[3,2-a]pyrimidin-5-one,
3-[2-[4-(6-fluoro-1,2-benzoisothiazol-3-yl)-1-piperidinyl]ethyl]-5H-7-methylthiazolo[3,2-a]pyrimidin-5-one,
3-[2-[4-(6-fluorobenzo[b]furan-3-yl)-1-piperidinyl]ethyl]-5H-7-methylthiazolo[3,2-a]pyrimidin-5-one,
3-[2-[4-(6-fluorobenzo[b]thiophen-3-yl)-1-piperidinyl]ethyl]-5H-7-methylthiazolo[3,2-a]pyrimidin-5-one,
3-[2-[4-(6-fluoro-1,2-benzisoxazol-3-yl)-1-piperidinyl]propyl]-5H-7-methylthiazolo[3,2-a]pyrimidin-5-one,
3-[2-[4-(6-fluorobenzo[b]furan-3-yl)-1-piperidinyl]ethyl]-5H-7-ethylthiazolo[3,2-a]pyrimidin-5-one,
3-[2-[4-(6-fluorobenzo[b]furan-3-yl)-1-piperidinyl]ethyl]-5H-7-propylthiazolo[3,2-a]pyrimidin-5-one,
3-[2-[4-(6-fluorobenzo[b]furan-3-yl)-1-piperidinyl]ethyl]-5H-7-isopropylthiazolo[3,2-a]pyrimidin- 5-one,
3-[2-[4-(6-fluoro-1,2-benzisoxazol-3-yl)-1-piperidinyl]ethyl]-5H-7-isopropylthiazolo[3,2-a]pyrimidin-5-one,
2,7-dimethyl-3-[2-[4-(6-fluoro-1,2-benzisoxazol-3-yl)-1-piperidinyl]ethyl]-5H-thiazolo[3,2-a]pyrimidin-5-one, ] 3-[2-[4-(6-fluoro-1,2-benzisoxazol*-*3*-*yl)-1-piperazinyl]methyl]-5H-thiazolo[2,3-b]pyrimidin-5-one, and
3-[2-[4-(6-fluoro-1,2-benzisoxazol-3-yl)-1-piperazinyl]methyl]-7H-thiazolo[2,3-b]pyrimidin-7-one.

The compounds according to the present invention may be formed into salts thereof. Such salts include medically acceptable non-toxic salts, for example, hydrogen halide salts, such as a hydrochloride salt, inorganic acid salts, such as a sulfuric acid salt, organic acid salts, such as succinic acid, citric acid, fumaric acid, and maleic acid salts, and amino acid salts, such as a glycine salt.

### Preparation of compounds represented by the formula (I)

The compounds according to the present invention may be prepared by the following methods.

### Method 1

Preferably, the compounds according to the present invention may be formed by Method 1 represented by the following scheme. This scheme shows a method for producing compounds where R¹ represents the group W-(CH₂)ₘ-. Compounds, wherein R² represents the group W-(CH₂)ₘ-, may be prepared in the same manner as described in the following scheme, except that the corresponding respective compounds are used as the starting compounds. 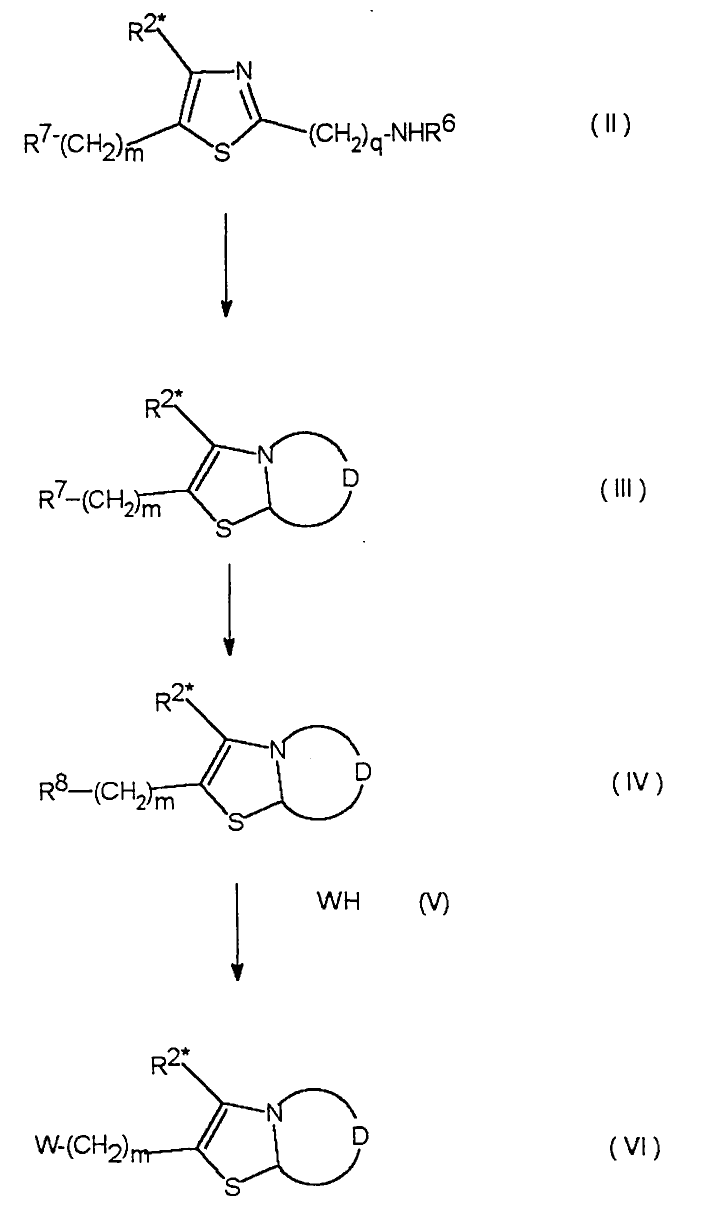 wherein
R^{2*} represents a hydrogen atom or optionally substituted lower alkyl,
R⁶ represents a hydrogen atom or amino, the amino being optionally protected by a protective group (for example, an acyl such as acetyl or benzoyl, benzyl, or trityl),
R⁷ represents a hydroxyl group which may be optionally protected by a protective group (for example, an ester, such as acetoxy or benzoyloxy, or an ether bond, such as tetrahydropyranyloxy and benzyloxy),
R⁸ represents a halogen atom or an eliminating group (for example, p-toluenesulfonyloxy or methanesulfonyloxy),
q is an integer of 0 or 1, and
W, m, and ring structure -D- are as defined above formula (I).

In the above scheme, at the outset, a compound of the formula (III) may be prepared from the corresponding aminothiazole derivative (II), for example, by modifying a method described in J. Heterocyclic Chem., 19, 343 (1982), ibid., 16, 1201 (1979), J. Med. Chem., 9, 545 (1966), and Chem. Commun., 1967, 991 (ring structure -D-: (a) or (b)) and a method described in Japanese Patent Laid-Open No. 5995/1979 and J. Heterocyclic Chem., 13, 291 (1976) (ring structure -D-: (c) or (d)).

Thereafter, the compound of the formula (III) may be treated by a conventional method (for example, a method wherein the compound of the formula (III), after the elimination of the protective group, is reacted with thionyl chloride, or a method wherein the compound of the formula (III) is reacted with p-toluenesulfonyl chloride) to give a compound of the formula (IV).

The compound of the formula (IV) may be reacted with a compound of the formula (V) in a solvent inert to the reaction (for example, anhydrous acetonitrile, dimethylformamide, or tetrahydrofuran) in the presence of an acid scavenger and, if necessary, a small amount of potassium iodide or sodium bromide at a reaction temperature of 20 to 110°C, preferably 40 to 80°C, for 2 to 72 hr, generally 5 to 24 hr, to give the compound of the formula (VI). Acid scavengers usable in this reaction include alkali carbonates, such as potassium carbonate and sodium bicarbonate, alkali metal compounds, such as sodium hydride, and organic amines, such as triethylamine.

### Method 2

Alternatively, the compounds of the formula (I) may be prepared by Method 2 represented by the following scheme. This scheme shows a method for producing compounds where R¹ represents the group W-(CH₂)ₘ-. Compounds, wherein R² represents the group W-(CH₂)ₘ*-*, may be prepared in the same manner as described in the following scheme, except that the corresponding respective compounds are used as the starting compounds. 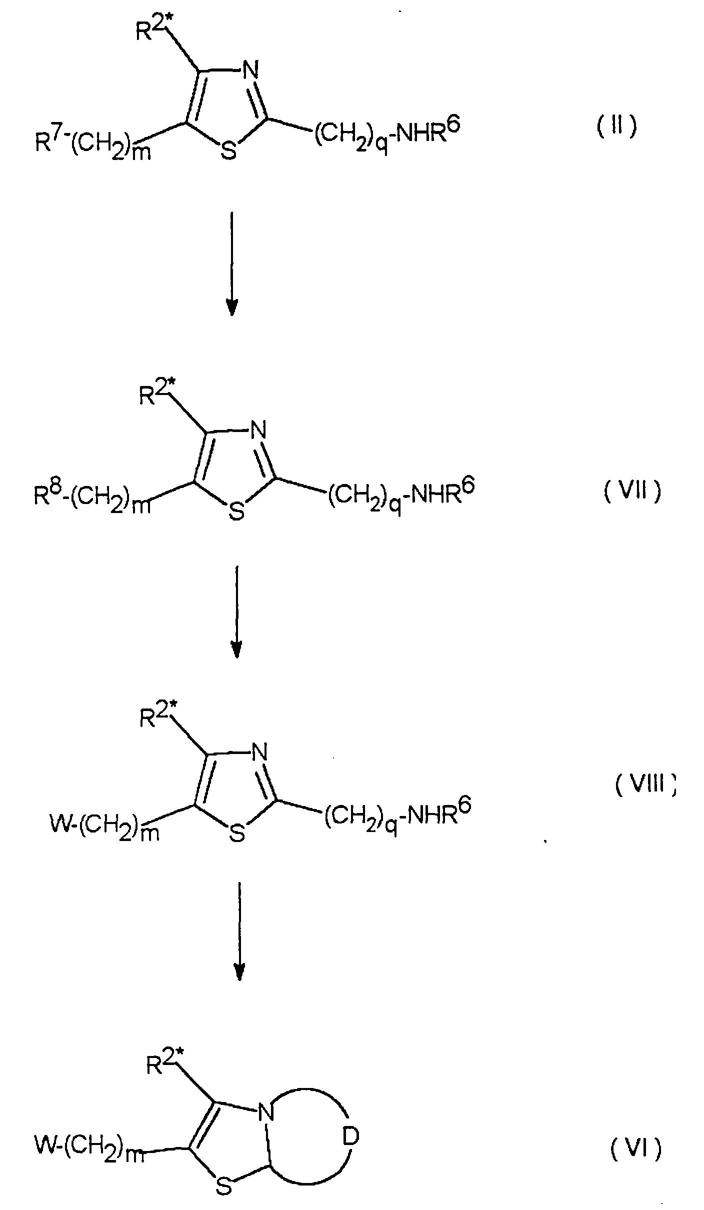 wherein
R^{2*}, R⁶, R⁷, R⁸, and q are as defined above in connection with Method 1, and
W, m, and ring structure -D- are as defined above in connection with the formula (I).

In the above scheme, the compound of the formula (VII) is prepared from the compound of the formula (II) under conditions as used in the reaction for the preparation of the compound (IV) from the compound of the formula (III) in Method 1.

The compound of the formula (VII) thus prepared may be reacted with the compound of the formula (V) under conditions as used in the reaction for the preparation of the compound (VI) from the compound of the formula (IV) in Method 1 to give a compound of the formula (VIII). Further, the compound of the formula (VIII) may be cyclized under conditions as used in the reaction for the preparation of the compound (III) from the compound of the formula (II) in Method 1 to give a compound of the formula (VI).

### Use of compound/pharmaceutical composition

The compounds of the formula (I) according to the present invention and pharmaceutically acceptable salts thereof have antimethamphetamine activity and, in addition, has high affinity for a D2 receptor, which is considered to be an important mechanism for antipsychoic activity and a 5-HT receptor, which is considered to be associated with central nervous-related psychoneurosis, including depression, anxiety neurosis and schizophrenia.

Therefore, the compounds of the present invention and pharmaceutically acceptable salts thereof may be used as psychotropic agents. Specifically, they may be used as therapeutic agents for mental disorders, such as neurosis, schizophrenia, and depression. The psychotropic agents referred herein include antischizophrenia agents, antianxiety agents, and antidepressants.

Pharmaceutical compositions comprising the compound according to the present invention as an active ingredient can be administered to human being and other animals by way of any one of the routes including oral and parenteral administration such as intravenous injection, intramuscular injection, subcutaneous administration, rectal administration or percutaneous administration. The pharmaceutical compositions comprising the compound according to the present invention as an active ingredient may therefore be formed into appropriate preparations corresponding to their route.

Specifically, tablets, capsules, powders, granules, syrups and the like may be mentioned as oral preparations, and injections, such as intravenous injections and intramuscular injections, rectal agents, oleosuppositories, aqueous suppositories and the like may be mentioned as parenteral preparations.

These preparations may be prepared by conventional methods using usually employed components, such as an excipient, an disintegrating agent, a binding agent, a lubricating agent, and a coloring matter.

Excipients usable herein include, for example, lactose, glucose, corn starch, sorbit, and crystalline cellulose; disintegrating agents usable herein include, for example, starch, sodium alginate, gelatin powder, calcium carbonate, calcium citrate, and dextrin; binding agents usable herein include, for example, dimethyl cellulose, polyvinyl alcohol, polyvinyl ether, methyl cellulose, ethyl cellulose, gum arabic, gelatin, hydroxypropyl cellulose, and polyvinyl pyrrolidone; and lubricating agents usable herein include, for example, talc, magnesium stearate, polyethylene glycol, and hydrogenated vegetable oil.

In the preparation of the injections, a buffer, a pH regulator, a stabilizing agent or the like may be, if necessary, added.

Although the content of the compound according to the present invention in the pharmaceutical composition depends on the preparations, it is generally in the range of from 0.1 to 50% by weight, preferably from about 0.1 to 20% by weight.

The dosage may be appropriately determined individually in view of the age, body weight and sex of patients, diseases and the seriousness of symptoms. In general, however, it ranges from 1 to 1,000 mg/adult/day, preferably from 1 to 300 mg/adult/day, which is administered once or in several portions a day.

### [EXAMPLES]

The present invention will be described in more detail with reference to the following examples, though it is not limited to these examples only.

### Example 1

### 2-Acetylamino-4-methyl-5-(2-chloroethyl)thiazole

Thionyl chloride (10 ml, 137 mmol) was added to 2-acetylamino-4-methyl-5-(2-hydroxyethyl)thiazole (8.0 g, 40 mmol), and the mixture was stirred at room temperature for 5 hr. Dichloromethane was added to the reaction solution, and the mixture was washed with water and dried over anhydrous magnesium sulfate. The solvent was removed by distillation under reduced pressure to give 8.7 g (yield 100%) of the title compound.
¹H NMR (CDCl₃)δ 2.32 (3H, s, CH₃), 2.37 (3H, s, CH₃), 3.15 (2H, t, J = 6.7 Hz, CH₂), 3.69 (2H, t, J = 6.7 Hz, CH₂); MW 218.71 (C₈H₁₁N₂₀SCl); mass spectrum EIMS, m/z 218 (M)⁺; m.p. 175-177°C.

### Example 2

### 2-Acetylamino-5-[2-[4-(6-fluoro-1,2-benzisoxazol-3-yl)-1-piperidinyl]ethyl]-4-methylthiazole

2-Acetylamino-5-(2-chloroethyl)-4-methylthiazole (1.0 g, 4.5 mmol), 4-(6-fluoro-1,2-benzisoxazol-3-yl)piperidine (1.0 g, 4.5 mmol), potassium iodide (150 mg, 0.90 mmol), and potassium carbonate (940 mg, 6.8 mmol) were stirred in acetonitrile (30 ml) with heating for 15 hr. The reaction solution was dissolved in a dichloromethane solution, and the mixture was washed with water and dried over anhydrous magnesium sulfate. The solvent was removed by distillation under reduced pressure to obtain an oil which was then purified by column chromatography on silica gel to give 906 mg (2.25 mmol, yield 50%) of the title compound.
¹H NMR (CDCl₃)δ 2.01-2.10 (2H, m, CH₂), 2.12-2.31 (4H, m, piperidine NCH₂), 2.23 (3H, s, CH₃), 2.26 (3H, s, CH₃), 2.62 (2H, t, J = 7.2 Hz, CH₂), 2.88 (2H, t, J = 7.2 Hz, CH₂), 3.06-3.16 (3H, m, piperidine CH-Ar, piperidine NCH₂), 7.09 (1H, ddd, J = 8.9, 8.6, 2.2 Hz, Ar), 7.24 (1H, dd, J = 8.6, 2.2 Hz, Ar), 7.85 (1H, dd, J = 8.6, 5.3 Hz, Ar); MW 402.49 (C₂₀H₂₃N₄O₂SF); mass spectrum EIMS, m/z 402 (M)⁺.

### Example 3

### 2-Amino-5-[2-[4-(1,2-benzoisothiazol-3-yl)-1-piperazinyl]ethyl]-4-methylthiazole

5 N HCl (10 ml) was added to a solution of 2-acetylamino-5-[2-[4-(1,2-benzoisothiazol-3-yl)-1-piperazinyl]ethyl]-4-methylthiazole (906 mg, 2.25 mmol) in methanol (10 ml), and the mixture was stirred with heating for 4 hr. The reaction solution was cooled and then adjusted to pH 9 by the addition of 5 N NaOH, followed by extraction with dichloromethane. The extract was dried over anhydrous magnesium sulfate, and the solvent was removed by distillation under reduced pressure. The residue was purified by column chromatography on silica gel to give 718 mg (1.99 mmol, yield 88%) of the title compound.
¹H NMR (CDCl₃)δ 2.04-2.18 (4H, m, CH₂), 2.15 (3H, s, CH₃), 2.20-2.28 (2H, m, piperidine NCH₂), 2.56 (2H, t, J = 7.4 Hz, CH₂), 2.78 (2H, t, J = 7.4 Hz, CH₂), 3.02-3.13 (3H, m, piperidine CH-Ar, piperidine NCH₂), 7.06 (1H, ddd, J = 9.0, 8.7, 2.1 Hz, Ar), 7.25 (1H, dd, J = 8.5, 2.1 Hz, Ar), 7.69 (1H, dd, J = 8.7, 5.1H, Ar); MW 360.444 (C₁₈H₂₁N₄OSF); mass spectrum SIMS, m/z 361 (M+1)⁺.

### Example 4

### 3,7-Dimethyl-2-[2-[4-(6-fluoro-1,2-benzisoxazol-3-yl)-1-piperidinyl]ethyl]-5H-thiazolo[3,2-a]pyrimidin-5-one

2-Amino-5-[2-[4-(6-fluoro-1,2-benzisoxazol-3-yl)-1-piperazinyl]ethyl]-4-methylthiazole (718 mg, 2.0 mmol) and ethyl acetoacetate (1.3 g, 10 mmol) were stirred with heating in polyphosphoric acid (9.0 g) at 130°C for 20 min. The reaction solution was poured into water, and the mixture was adjusted to pH 9 by the addition of 1 N NaOH and extracted with dichloroethane. The extract was dried over anhydrous magnesium sulfate, and the solvent was removed by distillation under reduced pressure to give an oil which was then purified by column chromatography on silica gel to give 457 mg (1.1 mmol, yield 54%) of the title compound.
¹H NMR (CDCl₃)δ 2.06-2.20 (4H, m, CH₂), 2.26-2.34 (2H, m, CH₂), 2.30 (3H, s, CH₃), 2.64 (2H, t, J = 6.4 Hz, CH₂), 2.76 (3H, s, CH₃), 2.84 (2H, t, J = 6.4 Hz, CH₂), 3.06-3.15 (1H, m, piperidine CH), 6.01 (1H, s, CH), 7.08 (1H, ddd, J = 9.0, 8.7, 2.1 Hz, Ar), 7.26 (1H, dd, J = 8.5, 2.1 Hz, Ar), 7.68 (1H, dd, J = 8.7, 4.9 Hz, Ar); MW 426.49 (C₂₂H₂₃N₄O₂SF); mass spectrum SIMS, m/z 427 (M+1)⁺; m.p. 182-186°C.

### Example 5

### 2-(2-Hydroxyethyl)-3,7-dimethyl-5H-thiazolo[3,2-a]pyrimidin-5-one

Ethyl acetoacetate (1.95 g, 15 mmol) and 5 g of polyphosphoric acid were added to 2*-*amino-4-methyl-5-(2-hydroxyethyl)thiazole (1.95 g, 10 mmol), and a reaction was allowed to proceed at 100 to 120°C for 4 hr and then at 150 to 160°C for one hr. Dichloromethane (30 ml) and 50 ml of water were added to the reaction solution to prepare a homogeneous solution which was then neutralized with a 5 N sodium hydroxide solution. The water layer was reextracted with 20 ml of dichloromethane, and the combined organic layer was washed with water and dried over anhydrous magnesium sulfate. The solvent was removed by distillation under reduced pressure, and the residue was subjected to separation and purification by column chromatography on silica gel to prepare the title compound.
¹H NMR (CDCl₃)δ 2.30 (3H, s, CH₃), 2.76 (3H, s, CH₃), 3.12 (2H, t, J = 6.7 Hz, CH₂), 3.70 (2H, t, J = 6.7 Hz, CH₂), 6.03 (1H, s, =CH); MW 224.28 (C₁₀H₁₂N₂O₂S); mass spectrum EIMS, m/z 224 (M)⁺.

### Example 6

### 2-(2-Chloroethyl)-5H-3,7-dimethylthiazolo[3,2-a]pyrimidin-5-one

Thionyl chloride (10 ml, 137 mmol) was added to 2-(2-hydroxyethyl)-5H-3,7-dimethylthiazolo[3,2-a]pyrimidin-7-one, and the mixture was stirred at room temperature for 5 hr. Dichloromethane was added to the reaction solution, and the mixture was washed with water and dried over anhydrous magnesium sulfate. The solvent was removed by distillation under reduced pressure to give 8.7 g (yield 77%) of the title compound.
¹H NMR (CDCl₃)δ 2.29 (3H, s, CH₃), 2.76 (3H, s, CH₃), 3.12 (2H, t, J = 6.7 Hz, CH₂), 3.70 (2H, t, J = 6.7 Hz, CH₂), 6.03 (1H, s, CH); MW 242.71 (C₁₀H₁₁N₂OSCl); mass spectrum EIMS, m/z 242 (M)⁺.

Further, the compound of Example 4 could be synthesized in the same manner as in Example 2, except that 2-(2-chloroethyl)-5H-3,7-dimethylthiazolo[3,2-a]pyrimidin-7-one was used instead of 2-acetylamino-5-(2-chloroethyl)-4-methylthiazole (yield 27%).

### Example 7

### 2-Acetylamino-5-[2-[4-(6-fluoro-1-benzothiophen-3-yl) -1-piperidinyl]ethyl]-4-methylthiazole

The title compound was synthesized in the same manner as in Example 2, except that 4-(6-fluoro-1-benzothiophen-3-yl)piperidine was used instead of 4-(6-fluoro-1,2-benzisoxazol-3-yl)piperidine.
¹H NMR (CDCl₃)δ 1.91 (2H, m, CH₂), 2.05 (2H, m, CH₂), 2.20-2.33 (2H, m, CH₂), 2.21 (3H, s, CH₃), 2.27 (3H, s, CH₃), 2.65 (2H, t, J = 7.2 Hz, CH₂), 2.86-2.95 (3H, m), 3.15 (2H, m, CH₂), 7.09 (1H, s, =CH), 7.14 (1H, ddd, J = 8.8, 8.8, 2.3 Hz, Ar), 7.54 (1H, dd, J = 8.8, 2.7c, Ar), 7.72 (1H, dd, J = 8.8, 5.0 Hz, Ar), 10-12 (1H, br, NH); MW 417.57 (C₂₁H₂₄N₃OS₂F); mass spectrum FDMS, m/z 418 (M+1)⁺.

### Example 8

### 3,7-Dimethyl-2-[2-[4-(6-fluoro-1-benzothiophen-3-yl)-1-piperidinyl]ethyl]-5H-thiazolo[3,2-a]pyrimidin-5-one

To a solution of 2-acetylamino-5-[2-[4-(6-fluoro-1-benzothiophen-3-yl)-1-piperidinyl]ethyl]-4-methylthiazole in ethanol was added 5 N HCl. The mixture was stirred with heating to give 2-amino-5-[2-[4-(6-fluoro-1-benzothiophen-3-yl)piperidinyl]ethyl]-4-methylthiazole. This compound (300 mg) and ethyl acetoacetate (0.43 ml) were stirred with heating in polyphosphoric acid (3.0 g) at 130°C for 20 min. The reaction solution was poured into water, and the mixture was adjusted to pH 9 by the addition of 1 N NaOH and extracted with dichloroethane. The extract was dried over anhydrous magnesium sulfate, and the solvent was removed by distillation under reduced pressure to give an oil which was then purified by column chromatography on silica gel to give 135 mg (yield 46%) of the title compound.
Free form: ¹H NMR (CDCl₃)δ 1.89 (2H, m, CH₂), 2.05 (2H, m, CH₂), 2.28 (2H, m, CH₂), 2.29 (3H, s, CH₃), 2.64 (2H, t, J = 6.3 Hz, CH₂), 2.76 (3H, s, CH₃), 2.84 (2H, t, J = 6.3 Hz, CH₂), 2.90 (1H, tt, J = 12, 3.5 Hz, CH), 3.10 (2H, m, CH₂), 6.00 (1H, d, J = 0.8 Hz, CH), 7.08 (1H, d, J = 0.8 Hz, Ar), 7.12 (1H, ddd, J = 9.0, 9.0, 2.3 Hz, Ar), 7.54 (1H, dd, J = 9.0, 2.4 Hz, Ar), 7.69 (1H, dd, J = 8.6, 5.1 Hz, Ar); MW 441.59 (C₂₃H₂₄N₃OS₂F); mass spectrum SIMS, m/z 442 (M+1)⁺.

The title compound in a free form thus obtained was dissolved in dioxane, and a solution of 4 N HCl in dioxane was added to the solution to give a hydrochloride.

Hydrochloride: m.p. 236-240°C (dec.).

### Example 9

### 2-(2-Hydroxyethyl)-3,6-dimethylimidazo[2,1-b]thiazole

Chloroacetone (1.91 ml, 24 mmol) was added to a solution of 2-amino-4-methyl-5-(2-hydroxyethyl)thiazole (3.16 g, 20 mmol) in isopropyl alcohol (100 ml), and a reaction was allowed to proceed under reflux for 3 hr. Thereafter, 1.91 ml of chloroacetone was further added thereto, and a reaction was allowed to proceed under reflux for 6 hr. The solvent was removed from the reaction solution under reduced pressure. Ethyl acetate and an 8% aqueous sodium bicarbonate solution were added to the residue, followed by separation, washing with water and drying over anhydrous magnesium sulfate. The solvent was removed by distillation under reduced pressure, and the residue was subjected to separation and purification by column chromatography on silica gel to give 1.65 g (yield 42%) of the title compound.
¹H NMR (CDCl₃)δ 2.25 (3H, s, CH₃), 2.37 (3H, d, J = 1.0 Hz, CH₃), 2.86 (2H, t, J = 6.1 Hz, CH₂), 3.83 (2H, t, J = 6.1 Hz, CH₂), 6.92 (1H, d, J = 1.0 Hz, =CH); MW 196.27 (C₉H₁₂N₂OS); mass spectrum EIMS, m/z 196 (M)⁺.

### Example 10

### 2-(2-Chloroethyl)-3,6-dimethylimidazo[2,1-b]thiazole

2-(2-Hydroxyethyl)-3,6-dimethylimidazo[2,1-b]thiazole (1.5 g, 7.64 mmol) was suspended in 15 ml of dichloromethane, 2.8 ml of thionyl chloride was added to the suspension, and the mixture was stirred at room temperature for 6.5 hr. The reaction solution was washed with water and dried over anhydrous sodium sulfate, and the solvent was removed by distillation under reduced pressure to give an oil which was then subjected to separation and purification by column chromatography on silica gel to give 1.32 g (yield 80%) of the title compound.
¹H NMR (CDCl₃)δ 2.31 (3H, s, CH₃), 2.35 (3H, s, CH₃), 3.11 (2H, t, J = 6.9 Hz, CH₂), 3.69 (2H, t, J = 6.9 Hz, CH₂), 7.03 (1H, s, Ar); MW 214.71 (C₉H₁₁N₂SCl); mass spectrum EIMS, m/z 214 (M)⁺, 216 (M+2)⁺.

### Example 11

### 3,6-Dimethyl-2-[2-[4-(6-fluoro-1,2-benzisoxazol-3-yl)-1-piperidinyl]ethyl]imidazo[2,1-b]thiazole

The title compound was synthesized in the same manner as in Example 2, except that 2-(2-chloroethyl)-3,6-dimethylimidazo[2,1-b]thiazole was used instead of 2-acetylamino-5-(2-chloroethyl)-4*-*methylthiazole (yield 55%).
Free form: ¹H NMR (CDCl₃)δ 2.02-2.19 (4H, m, CH₂), 2.23-2.31 (2H, m, CH₂), 2.28 (3H, s, CH₃), 2.35 (3H, s, CH₃), 2.64 (2H, t, J = 6.9 Hz, CH₂), 2.86 (2H, t, J = 6.9 Hz, CH₂), 3.06-3.15 (3H, m, CH₂ and piperidine CH), 7.00 (1H, s, Ar), 7.07 (1H, ddd, J = 9.0, 8.7, 2.3 Hz, Ar), 7.22 (1H, dd, J = 8.7, 2.3 Hz, Ar), 7.71 (1H, dd, J = 8.7, 5.1 Hz, Ar); MW 398.48 (C₂₁H₂₃N₄OSF); mass spectrum EIMS, m/z 398 (M)⁺.

The title compound in a free form thus obtained was dissolved in dioxane, and a solution of 4 N HCl in dioxane was added to the solution to give a hydrochloride.
Hydrochloride: MW 434.94(C₂₁H₂₄N₄OSClF); mass spectrum EIMS, m/z 398(M+1-HCl)⁺; m.p. 210°C (dec. ).

### Example 12

### 3,6-Dimethyl-2-[2-[4-(6-fluoro-1-benzothiophen-3-yl)-1-piperidinyl]ethyl]imidazo[2,1-b]thiazole

The title compound was synthesized in the same manner as in Example 2, except that 4-(6-fluoro-1-benzothiophen-3-yl)piperidine was used instead of 4-(6-fluoro-1,2-benzisoxazol-3-yl)piperidine and, further, 2-(2-chloroethyl)-3,6-dimethylimidazo[2,1-b]thiazole was used instead of 5-(2-chloroethyl)-2-acetylamino-4-methylthiazole (yield 55%).
Free form: ¹H NMR (CDCl₃)δ 1.84-1.93 (2H, m, CH₂), 2.00-2.08 (2H, m, CH₂), 2.21-2.26 (2H, m, CH₂), 2.28 (3H, s, CH₃), 2.34 (3H, d, J = 1.0 Hz, CH₃), 2.64 (2H, t, J = 7.2 Hz, CH₂), 2.87 (2H, t, J = 7.2 Hz, CH₂), 2.87-2.94 (1H, m, piperidine CH), 3.07-3.14 (2H, m, CH₂), 7.00 (1H, d, J = 1.0 Hz, Ar), 7.07 (1H, s, Ar), 7.13 (1H, ddd, J = 9.0, 8.7, 2.0 Hz, Ar), 7.54 (1H, dd, J = 8.7, 2.3 Hz, Ar), 7.74 (1H, dd, J = 9.0 5.1 Hz, Ar); MW 413.55 (C₂₂H₂₄N₃S₂F); mass spectrum EIMS, m/z 413 (M+1)⁺.

The title compound in a free form thus obtained was dissolved in dioxane, and a solution of 4 N HCl in dioxane was added to the solution to give a hydrochloride.
Hydrochloride: MW 450.01 (C₂₂H₂₅N₃S₂ClF); mass spectrum SIMS, m/z 414 (M+1-HCl)⁺; m.p. 230°C (dec.).

### Example 13

### 2-Acetoacetylamino-5-(2-acetoacetoxyethyl)-4-methylthiazole

2-Amino-4-methyl-5-(2-hydroxyethyl)thiazole (500 mg, 3.16 mmol) was suspended in 10 ml of toluene, diketene (0.53 ml, 7 mmol) was added to the suspension, and the mixture was stirred in an argon atmosphere at 80°C for 3.5 hr. After the reaction, toluene was removed by distillation under reduced pressure. The residue was purified by column chromatography on silica gel (dichloromethane : methanol = 50 : 1) to give 794 mg (yield 85%) of the title compound.
¹H NMR (CDCl₃)δ 2.26 (3H, s, CH₃), 2.27 (3H, s, CH₃), 2.33 (3H, s, CH₃), 3.03 (2H, t, J = 6.9 Hz, CH₂), 3.48 (2H, s, CH₂), 3.66 (2H, s, CH₂), 4.29 (2H, m, CH₂), 10.45 (1H, br s, NH); MW 326.35 (C₁₄H₁₈N₂O₅S); mass spectrum EIMS, m/z 326 (M)⁺ ; m.p. 128-130°C.

### Example 14

### 2-(2-Hydroxyethyl)-7H-3,5-dimethylthiazolo[3,2-a]pyrimidin-7-one

Concentrated sulfuric acid (1.5 ml) was added to 2-acetoacetylamino-5-(2-acetoacetoxyethyl)-4-methylthiazole (326 mg, 1 mmol), and the mixture was stirred at 60°C for 2.5 hr. The reaction solution was poured into ice, and the mixture was adjusted to pH 11 by the addition of a 5 N aqueous NaOH solution and extracted with dichloromethane. The extract was dried over sodium sulfate, and the dichloromethane was removed by distillation under reduced pressure to give 160 mg (yield 70%) of the title compound.
¹H NMR (CDCl₃)δ 2.27 (3H, s, CH₃), 2.72 (3H, s, CH₃), 2.81 (1H, br s, OH), 2.88 (2H, t, J = 6.1 Hz, CH₂), 2.83-2.70 (2H, m, CH₂), 5.96 (1H, s, CH); MW 224.27 (C₁₀H₁₂N₂O₂S); mass spectrum EIMS, m/z 224 (M)⁺; m.p. 89-91°C.

### Example 15

### 2-(2-Chloroethyl)-7H-3,5-dimethylthiazolo[3,2-a]pyrimidin-7-one

The title compound was synthesized in the same manner as in Example 9, except that 2-(2-hydroxyethyl)-7H-3,5-dimethylthiazolo[3,2-a]pyrimidin-7-one was used instead of 2-(2-hydroxyethyl)-3,6-dimethylimidazo[2,1-b]thiazole (yield 88%).
¹H NMR (CDCl₃)δ 2.30 (3H, s, CH₃), 2.76 (3H, s, CH₃), 3.12 (2H, t, J = 6.7 Hz, CH₂), 3.70 (2H, t, J = 6.7 Hz, CH₂), 6.04 (1H, s, CH); MW 242.71 (C₁₀H₁₁N₂OSCl); mass spectrum EIMS, m/z 242 (M)⁺, 244 (M+2)⁺; m.p. 88-90°C.

### Example 16

### 3,5-Dimethyl-2-[2-[4-(6-fluoro-1,2-benzisoxazol-3-yl)-1-piperidinyl]ethyl]-7H-thiazolo[3,2-a]pyrimidin-7-one

The title compound was synthesized in the same manner as in Example 2, except that 2-(2-chloroethyl)-7H-3,5-dimethylthiazolo[3,2-a]pyrimidin-7-one was used instead of 2-acetylamino-5-(2-chloroethyl)-4-methylthiazole (yield 28%).
¹H NMR (CDCl₃)δ 2.05-2.20 (4H, m, CH₂), 2.25-2.35 (2H, m, CH₂), 2.30 (3H, s, CH₃), 2.65 (2H, t, J = 6.4 Hz, CH₂), 2.76 (3H, s, CH₃), 2.84 (2H, t, J = 6.4 Hz, CH₂), 3.04-3.16 (3H, m, CH₂, piperidine CH), 6.00 (1H, s, CH), 7.08 (1H, ddd, J = 9.0, 8.7, 2.1 Hz, Ar), 7.25 (1H, dd, J = 8.5, 2.1 Hz, Ar), 7.69 (1H, dd, J = 8.7, 4.9 Hz, Ar); MW 426.49 (C₂₂H₂₃N₄O₂SF); mass spectrum SIMS, m/z 428 (M+2)⁺ ; m.p. 180-182°C.

### Example 17

### 3-Ethoxycarbonylethyl-5H-7-methylthiazolo[3,2-a]pyrimidin-5-one

2-Amino-4-ethoxycarbonylethylthiazole (3.724 g, 20 mmol) and ethyl acetoacetate (3.8 ml, 30 mmol) were stirred in 15 g of polyphosphoric acid at 130°C for 45 min. The reaction solution was poured into water, and the mixture was adjusted to pH 9 by the addition of a 1 N aqueous NaOH solution and extracted with dichloromethane. The extract was dried over anhydrous sodium sulfate, and the solvent was removed by distillation under reduced pressure to give an oil which was then purified by column chromatography on silica gel (dichloromethane : methanol = 50 : 1) to give 3.92 g (yield 78%) of the title compound.
¹H NMR (CDCl₃)δ 1.28 (3H, t, J = 7.2 Hz, CH₃), 2.30 (3H, s, CH₃), 4.12 (2H, q, J = 7.2 Hz, CH₂), 4.21 (2H, q, J = 7.2 Hz, CH₂), 6.04 (1H, s, = CH), 6.62 (1H, s, = CH); MW 252.28 (C₁₁H₁₂N₂O₃S); mass spectrum EIMS, m/z 252 (M)⁺; m.p. 100-102°C.

### Example 18

### 3-(2-Hydroxyethyl)-5H-7-methylthiazolo[3,2-a]pyrimidin-5-one

3*-*Ethoxycarbonylethyl-5H-7-methylthiazolo[3,2-a]pyrimidin-5-one (1.0 g, 3.96 mmol) was dissolved in 20 ml of tetrahydrofuran, lithium aluminum hydride (150 mg, 3.96 mmol) was added to the solution, and the mixture was stirred in an argon atmosphere at 0°C for one hr. Water (1 ml) was dropwise added to the reaction solution, and the mixture was stirred for 30 min. Tetrahydrofuran was removed by distillation under reduced pressure. The residue was dissolved in ethyl acetate, the solution was washed with water and dried over anhydrous sodium sulfate, and the solvent was removed by distillation under reduced pressure. The residue was purified by column chromatography on silica gel (hexane : ethyl acetate = 2 : 5) to give 250 mg (yield 30%) of the title compound.
¹H NMR (CDCl₃)δ 2.31 (3H, s, CH₃), 3.02 (H, br s, OH), 3.47 (2H, t, J = 5.8 Hz, CH₂), 3.93 (2H, t, J = 5.8 Hz, CH₂), 6.06 (1H, s, = CH), 6.61 (1H, s, = CH); MW 210.24 (C₉H₁₀N₂O₂S); mass spectrum EIMS, m/z 210 (M)⁺; m.p. 113-115°C.

### Example 19

### 3-(2-Chloroethyl)-5H-7-methylthiazolo[3,2-a]pyrimidin-5-one

The title compound was synthesized in the same manner as in Example 9, except that 3-(2-hydroxyethyl)-5H-7-methylthiazolo[3,2-a]pyrimidin-5-one was used instead of 2-(2-hydroxyethyl)-3,6-dimethylimidazo[2,1-b]thiazole (yield 67%).
¹H NMR (CDCl₃)δ 2.34 (3H, s, CH₃), 3.62 (2H, t, J = 6.2 Hz, CH₂), 3.89 (2H, t, J = 6.2 Hz, CH₂), 6.08 (1H, s, = CH), 6.63 (1H, s, = CH); MW 228.69 (C₉H₉N₂O₂S); mass spectrum EIMS, m/z 228 (M)⁺, 230 (M+2)⁺; m.p. 135-137°C.

### Example 20

### 3-[2-[4-(6-Fluoro-1,2-benzisoxazol-3-yl)-1-piperidinyl]ethyl]-5H-7-methylthiazolo[3,2-a]pyrimidin-5-one

The title compound was synthesized in the same manner as in Example 2, except that 3-(2-chloroethyl)-5H-7-methylthiazolo[3,2-a]pyrimidin-5-one was used instead of 2-acetylamino-5-(2-chloroethyl)-4-methylthiazole (yield 60%).
¹H NMR (CDCl₃)δ 2.00-2.12 (4H, m, CH₂), 2.25-2.35 (2H, m, CH₂), 2.33 (3H, s, CH₃), 2.80 (2H, t, J = 7.2 Hz, CH₂), 3.03-3.16 (3H, m, CH₂, piperidine CH), 3.47 (2H, t, J = 7.2 Hz, CH₂), 6.06 (1H, s, =CH), 6.64 (1H, s, = CH), 7.06 (1H, ddd, J = 8.9, 8.6, 2.2 Hz, Ar), 7.24 (1H, dd, J = 8.6, 2.2 Hz, Ar), 7.68 (1H, dd, J = 8.9, 5.0 Hz, Ar); MW 412.46 (C₂₁H₂₁N₄O₂SF); mass spectrum EIMS, m/z 412 (M)⁺; m.p. 189-191°C.

### Example 21

### 6-Ethoxycarbonyl-2-(2-hydroxyethyl)-5H-3-methylthiazolo[3,2-a]pyrimidin-5-one

2-Amino-4-methyl-5-(2-hydroxyethyl)thiazole (8.5 g, 54 mmol) and diethyl ethoxyethylenemalonate (11.6 g, 54 mmol) were heated at 120°C for one hr to give 13.9 g (42.5 mmol) of an adduct and 236 mg (0.93 mmol, yield 1.7%) of a cyclization product as a contemplated compound.

The adduct thus obtained was reacted by the following method to give the contemplated cyclization product. NaH (290 mg, 12 mmol) was added to a solution of the adduct (3.31 g, 10 mmol) in THF (30 ml), and the mixture was heated under reflux for 7 hr. The solvent was removed by distillation under reduced pressure, and the residue was then purified by column chromatography on silica gel to give 341 mg (1.34 mmol, yield 13%) of the contemplated cyclization compound. Further, 2.8 g (8.5 mmol, yield 85%) of the adduct as the starting compound was recovered.

### Adduct:

¹H NMR (CDCl₃)δ 1.32 (3H, t, J = 7.2 Hz, CH₂CH₃), 1.37 (3H, t, J = 7.2 Hz, CH₂CH₃)2.26 (3H, s, CH₃)2.92 (2H, t, J = 6.10 Hz, CH₂), 3.81 (2H, t, J = 6.1 Hz, CH₂), 4.24 (2H, q, J = 7.2 Hz, CH₂CH₃), 4.30 (2H, q, J = 7.2 Hz, CH₂CH₃), 8.60 (1H, d, J = 12.2 Hz, CH=C); MW 328.38 (C₁₄H₂₀N₂O₅S); mass spectrum EIMS, m/z 328 (M)⁺.

### Cyclization product:

¹H NMR (CDCl₃)δ 1.38 (3H, t, J = 7.2 Hz, CH₂CH₃)2.81 (3H, s, CH₃)2.95 (2H, t, J = 5.9 Hz, CH₂), 3.91 (2H, t, J = 5.9 Hz, CH₂), , 4.37 (2H, q, J = 7.2 Hz, CH₂CH₃), 8.64 (1H, s, CH = C); MW 282.31 (C₁₂H₁₄N₂O₄S) mass spectrum EIMS, m/z 282 (M)⁺.

### Example 22

### 6-Ethoxycarbonyl-2-(2-chloroethyl)-5H-3-methylthiazolo[3,2-a]pyrimidin-5-one

Thionyl chloride (1 ml) was added to a solution of the cyclization product (236 mg, 0.84 mmol) of Example 20 in dichloromethane (1 ml), and the mixture was stirred at room temperature for 15 hr. The reaction solution was subjected to distillation under reduced pressure, and the residue was purified by column chromatography on silica gel to give 211 mg (0.70 mmol, yield 84%) of the contemplated chloro compound.

### Example 23

### 6-Ethoxycarbonyl-2-[2-[4-(6-fluoro-1,2-benzisoxazol -3-yl)-1-piperidinyl]ethyl]-5H-3-methylthiazolo[3,2-a]pyrimidin-5-one

The title compound was synthesized in the same manner as in Example 2, except that the cyclized chloro compound prepared in Example 21 was used instead of 2-acetylamino-4-methyl-5-(2-chloroethyl)thiazole (yield 18%).
¹H NMR (CDCl₃)δ 1.39 (3H, t, J = 7.2 Hz, CH₂CH₃)2.07-2.21 (4H, m, piperidine CH₂), 2.30-2.38 (2H, m, piperidine NCH₂), 2.67 (2H, t, J = 6.0 Hz, CH₂)2.83 (3H, s, CH₃), 2.88 (2H, t, J = 6.0 Hz, CH₂), 3.06-3.18 (3H, m, piperidine NCH₂, piperidine CH-Ar), 4.38 (2H, q, J = 7.2 Hz, CH₂CH₃), 7.08 (1H, ddd, J = 9.0, 8.7, 2.1 Hz, Ar), 7.26 (1H, dd, J = 8.2, 2.1 Hz, Ar), 7.68 (1H, dd, J = 8.7, 5.1 Hz, Ar); MW 484.53 (C₂₄H₂₅N₄O₄SF); mass spectrum EIMS, m/z 484 (M)⁺.

### Example 24

### 6-Ethoxycarbonyl-2-[2-[4-(1,2-benzoisothiazol-3-yl)-1-piperazinyl]ethyl]-5H-3-methylthiazolo[3,2-a]pyrimidin-5-one

The cyclized chloro compound (205 mg, 0.68 mmol) prepared in Example 21, 3-(1-piperazinyl)-1,2-benzoisothiazole (150 mg, 0.68 mmol), potassium iodide (23 mg, 0.14 mmol), and potassium carbonate (141 mg, 1.0 mmol) were stirred with heating in acetonitrile (6 ml) for 15 hr. The reaction solution was dissolved in a dichloromethane solution, the solution was washed with water and dried over anhydrous magnesium sulfate, and the solvent was removed by distillation under reduced pressure to obtain an oil which was then subjected to separation by column chromatography on silica gel to give 74 mg (0.15 mmol, yield 23%) of the title compound.
¹H NMR (CDCl₃) δ 1.38 (3H, t, J = 7.2 Hz, CH₂CH₃), 2.72 (2H, t, J = 6.2 Hz, CH₂), 2.79 (2H, t, J = 4.9 Hz, piperazine CH₂), 2.83 (3H, s, CH₃), 2.90 (2H, t, J = 6.2 Hz, CH₂), 3.63 (2H, t, J = 4.9 Hz, piperazine CH₂), 4.37 (2H, q, J = 7.2 Hz, CH₂CH₃)7.37 (1H, dd, J = 8.2, 7.2 Hz, Ar), 7.48 (1H, dd, J = 8.2, 7.2 Hz, Ar), 7.82 (1H, d, J = 8.2 Hz, Ar) 7.90 (1H, d, J = 8.2 Hz, Ar) 8.64 (1H, s, C = CH); MW 483.59 (C₂₃H₂₅N₅O₃S₂); mass spectrum EIMS, m/z 483 (M)⁺.

### Example 25

### 3-[2-[4-(6-Fluoro-1,2-benzisoxazol-3-yl)-1-piperidinyl]ethyl]-6-methylimidazo[2,1-b]thiazole

The title compound was synthesized in the same manner as in Example 2, except that 3-(2-chloroethyl)-6-methylimidazo[2,1-b]thiazole was used instead of 2-acetylamino-5-(2-chloroethyl)-4-methylthiazole (yield 29%).
Free form: ¹H NMR (CDCl₃)δ 2.06-2.14 (4H, m, CH₂), 2.22-2.32 (2H, m, CH₂), 2.37 (3H, s, CH₃), 2.76-2.82 (2H, m, CH₂) 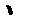 2.89-2.94 (2H, m, CH₂), 3.06-3.15 (3H, m, CH₂ 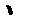 piperidine CH), 6.47 (1H, s, = CH), 7.07 (1H, dt, J = 8.9, 2.2 Hz, Ar), 7.16 (1H, d, J = 0.8 Hz, = CH), 7.25 (1H, dd, J = 8.6, 2.2 Hz, Ar), 7.69 (1H, dd, J = 8.9, 5.0 Hz, Ar); MW 384.45 (C₂₀H₂₁N₄OSF); mass spectrum EIMS, m/z 384 (M)⁺.

The title compound in a free form thus obtained was dissolved in dioxane, and a solution of 4 N HCl in dioxane was added to the solution to give a hydrochloride.
Hydrochloride: MW 457.37 (C₂₀H₂₂N₄SOClF); mass spectrum SIMS, m/z 385 (M+1-HCl)⁺; m.p. 215°C (dec.).

### Example 26

### 3-[2-[4-(6-Fluoro-1,2-benzisoxazol-3-yl)-1-piperidinyl]ethyl]-2-methylimidazo[2,1-b]thiazole

The title compound was synthesized in the same manner as in Example 2, except that 3-(2-chloroethyl)-2-methylimidazo[2,1-b]thiazole was used instead of 2-acetylamino-5-(2-chloroethyl)-4-methylthiazole.
¹H NMR (CDCl₃)δ 2.03-2.12 (4H, m, CH₂), 2.20-2.31 (2H, m, CH₂), 2.37 (3H, s, CH₃), 2.66 (2H, t, J = 7.3, 8.0 Hz, CH₂), 2.95 (2H, t, J = 7.3, 8.0 Hz, CH₂), 3.06-3.12 (3H, m, CH₂ and CH), 7.07 (1H, ddd, J = 2.3, 8.8, 8.9 Hz, Ar), 7.25 (1H, dd, J = 2.3, 8.8 Hz, Ar), 7.30 (1H, d, J = 1.5 Hz, Ar), 7.36 (1H, d, J = 1.5 Hz, Ar), 7.68 (1H, dd, J = 5.3, 8.8 Hz, Ar); MW 384.47 (C₂₀H₂₁N₄OSF); mass spectrum EIMS m/z 384 (M⁺).

### Example 27

### 3-[2-[4-(6-Fluoro-1,2-benzisoxazol-3-yl)-1-piperidinyl]ethyl]-2,6-dimethylimidazo[2,1-b]thiazole

The title compound was synthesized in the same manner as in Example 2, except that 3-(2-chloroethyl)-2,6-dimethylimidazo[2,1-b]thiazole was used instead of 2-acetylamino-5-(2-chloroethyl)-4-methylthiazole.
¹H NMR (CDCl₃)δ 2.08-2.12 (4H, m, CH₂), 2.27 (2H, m, CH₂), 2.34 (3H, s, CH₃), 2.35 (3H, d, J = 0.8 Hz, CH₃), 2.64 (2H, m, CH₂), 2.90 (2H, m, CH₂), 3.10 (3H, m, CH₂ and CH), 7.05 (1H, dd, J = 2.2, 8.8 Hz, Ar), 7.08 (1H, m, Ar), 7.26 (1H, dd, J = 8.8, 2.2 Hz, Ar), 7.68 (1H, dd, J = 5.0, 8.6 Hz, Ar); MW 398.55 (C₂₁H₂₃N₄OSF); mass spectrum EIMS m/z 398 (M⁺).

### Example 28

### 3-[2-[4-(6-Fluorobenzo[b]furan-3-yl)-1-piperidinyl]ethyl]-6-isopropylimidazo[2,1-b]thiazole

The title compound was synthesized in the same manner as in Example 2, except that 3-(2-chloroethyl)-6-isopropylimidazo[2,1-b]thiazole was used instead of 2-acetylamino-5-(2-chloroethyl)-4-methylthiazole and, further, 4-(6-fluorobenzo[b]furan-3-yl)piperazine was used instead of 4-(6-fluoro-1,2-benzisoxazol-3-yl)piperidine.
¹H NMR (CDCl₃)δ 1.32 (6H, d, J = 6.8 Hz, CH (CH₃)2), 1.83 (2H, m, CH₂), 2.08 (2H, m, CH₂), 2.23 (2H, m, CH₂), 2.74-2.80 (3H, m, CH₃ and CH₂), 2.92 (2H, m, CH₂), 3.01 (1H, m, CH), 3.10 (2H, m, CH₂), 6.45 (1H, s, CH), 7.01 (1H, dddd, J = 2.3, 8.4, 9.6, 0.8 Hz, Ar), 7.14 (1H, d, J = 0.8 Hz, Ar), 7.18 (1H, dd, J = 2.3, 9.2 Hz, Ar), 7.39 (1H, d, J = 1.2 Hz, Ar), 7.50 (1H, dd, J = 5.3, 8.4 Hz, Ar); MW 411.53 (C₂₃H₂₆N₃OSF); mass spectrum EIMS m/z 411 (M⁺).

### Example 29

### 3-[2-[4-(6-Fluoro-1,2-benzisoxazol-3-yl)-1-piperidinyl]ethyl]-6-isopropyl-2-methylimidazo[2,1-b]thiazole

The title compound was synthesized in the same manner as in Example 2, except that 6-isopropyl-3-(2-methanesulfonyloxyethyl)-2-methylimidazo[2,1-b]thiazole was used instead of 2-acetylamino-5-(2-chloroethyl)-4-methylthiazole.
¹H NMR (CDCl₃)δ 1.31 (6H, d, J = 7.0 Hz, CH (CH₃)2), 2.00-2.18 (4H, m, CH₂), 2.28 (2H, m, CH₂), 2.34 (3H, s, CH₃), 2.65 (2H, m, CH₂), 2.91 (2H, m, CH₂), 2.99 (1H, m, piperidine CH), 3.00-3.20 (3H, m, CH and CH₂), 7.02-7.12 (2H, m, Ar), 7.26 (1H, dd, J = 8.7, 2.2 Hz, Ar), 7.68 (1H, dd, J = 8.8, 5.1 Hz, Ar); MW 426.53 (C₂₃H₂₇N₄OSF); mass spectrum EIMS, m/z 426 (M)⁺.

### Example 30

### 3-[2-[4-(6-Fluorobenzo[b]furan-3-yl)-1-piperidinyl]ethyl]-6-isopropyl-2-methylimidazo[2,1-b]thiazole

The title compound was synthesized in the same manner as in Example 2, except that 6-isopropyl-3-(2-methanesulfonyloxyethyl)-2-methylimidazo[2,1-b]thiazole was used instead of 2-acetylamino-5-(2-chloroethyl)-4-methylthiazole and, further, 4-(6-fluorobenzo[b]furan-3-yl)piperazine was used instead of 4-(6-fluoro-1,2-benzisoxazol-3-yl)piperidine.
¹H NMR (CDCl₃)δ 1.31 (6H, d, J = 6.7 Hz, CH (CH₃)2), 1.82 (2H, m, CH₂), 2.20-2.13 (2H, m, CH₂), 2.24 (2H, m, CH₂), 2.33 (3H, s, CH₃), 2.63 (2H, m, CH₂), 2.74 (1H, m, piperidine CH), 2.91 (2H, m, CH₂), 2.99 (1H, septet, J = 6.7 Hz, CH-iPr), 3.11 (2H, m, CH₂), 7.00 (1H, ddd, J = 9.5, 8.6, 2.2 Hz, Ar), 7.06 (1H, d, J = 0.8 Hz, Ar), 7.19 (1H, dd, J = 9.1, 2.2 Hz, Ar), 7.39 (1H, d, J = 0.8 Hz, Ar), 7.50 (1H, dd, J = 8.6, 5.3 Hz, Ar); MW 425.54 (C₂₄H₂₈N₃OSF); mass spectrum EIMS, m/z 425 (M)⁺.

### Example 31

### 3-[2-[4-(1,2-Benzoisothiazol-3-yl)-1-piperazinyl]ethyl]-6-isopropyl-2-methylimidazo[2,1-b]thiazole

The title compound was synthesized in the same manner as in Example 2, except that 6-isopropyl-3-(2-methanesulfonyloxyethyl)-2*-*methylimidazo[2,1-b]thiazole was used instead of 2*-*acetylamino-5-(2-chloroethyl)-4-methylthiazole and, further, 1-(1,2-benzoisothiazol-3-yl)piperazine was used instead of 4-(6-fluoro-1,2-benzisoxazol-3-yl)piperidine.
¹H NMR (CDCl₃)δ 1.31 (6H, d, J = 6.9 Hz, -CH (CH₃)2), 2.34 (3H, s, CH₃), 2.69 (2H, m, CH₂), 2.77 (4H, t, J = 4.9 Hz, CH₂), 2.93 (2H, m, CH₂), 2.99 (1H, septet, J = 6.9 Hz, CH-iPr), 3.60 (4H, t, J = 4.9 Hz, CH₂), 7.07 (1H, d, J = 0.8 Hz, Ar), 7.36 (1H, ddd, J = 8.2, 7.0, 1.1 Hz, Ar), 7.48 (1H, ddd, J = 8.2, 7.0, 1.1 Hz, Ar), 7.82 (1H, d, J = 8.2 Hz, Ar), 7.91 (1H, d, J = 8.2 Hz, Ar); MW 425.60 (C₂₂H₂₇N₅S₂); mass spectrum EIMS, m/z 425 (M)⁺.

### Example 32

### 3-[2-[4-(1,2-Benzoisothiazol-3-yl)-1-piperazinyl]ethyl]-5H-7-methylthiazolo[3,2-a]pyrimidin-5-one

The title compound was synthesized in the same manner as in Example 2, except that 3-(2-chloroethyl)-5H-7-methylthiazolo[3,2-a]pyrimidin-5-one was used instead of 2-acetylamino-5-(2-chloroethyl)-4-methylthiazole, and further, 1-(1,2-benzoisothiazol-3-yl)piperazine was used instead of 4-(6-fluoro-1,2-benzisoxazol-3-yl)piperidine (yield 49%).
¹H NMR (CDCl₃)δ 2, 33 (3H, s, CH₃), 2.78 (4H, t, J = 5.0 Hz, CH₂), 2.84 (2H, t, J = 7.6 Hz, CH₂), 3.49 (2H, t, J = 7.6 Hz, CH₂), 3.56 (4H, t, J = 5.0 Hz, CH₂), 6.07 (1H, s, Ar), 6.65 (1H, s, Ar), 7.36 (1H, ddd, J = 8.0, 7.0, 1.1 Hz, Ar), 7.47 (1H, ddd, J = 8.0, 7.0, 1.1 Hz, Ar), 7.81 (1H, dt, J = 8.0, 1.1 Hz, Ar), 7.91 (1H, dt, J = 8.0, 1.1 Hz, Ar); MW 411.53 (C₂₀H₂₁N₅OS₂); mass spectrum SIMS, m/z 411 (M)⁺.

### Example 33

### 3-[2-[4-(6-Fluoro-1,2-benzoisothiazol-3-yl)-1-piperidinyl]ethyl]-5H-7-methylthiazolo[3,2-a]pyrimidin-5-one

The title compound was synthesized in the same manner as in Example 2, except that 3-(2-chloroethyl)-5H-7-methylthiazolo[3,2-a]pyrimidin-5-one was used instead of 2-acetylamino-5-(2-chloroethyl)-4-methylthiazole and, further, 4-(6-fluoro-1,2-benzoisothiazol-3-yl)piperidine was used instead of 4-(6-fluoro-1,2-benzisoxazol-3-yl)piperidine (yield 56%).
¹H NMR (CDCl₃)δ 2.0-2.15 (4H, m, CH₂), 2.25-2.37 (2H, m, CH₂), 2.33 (3H, s, CH₃), 2.81 (2H, t, J = 7.1 Hz, CH₂), 3.1-3.26 (3H, m, CH₂ and piperidine CH), 3.49 (2H, t, J = 7.1 Hz, CH₂), 6.06 (1H, s, = CH), 6.70 (1H, s, = CH), 7.17 (1H, dt, J = 8.7, 2.3 Hz, Ar), 7.57 (1H, dd, J = 8.2, 2.3 Hz, Ar), 7.93 (1H, dd, J = 8.7, 4.9 Hz, Ar); MW 428.52 (C₂₁H₂₁N₄O₂SF); mass spectrum SIMS, m/z 426 (M-2)⁺; m.p. 180-183°C.

### Example 34

### 3-[2-[4-(6-Fluorobenzo[b]furan-3-yl)-1-piperidinyl]ethyl]-5H-7-methylthiazolo[3,2-a]pyrimidin-5-one

The title compound was synthesized in the same manner as in Example 2, except that 3-(2-chloroethyl)-5H-7-methylthiazolo[3,2-a]pyrimidin-5-one was used instead of 2-acetylamino-5-(2-chloroethyl)-4-methylthiazole and, further, 4-(6-fluorobenzo[b]furan-3-yl)piperidine was used instead of 4-(6-fluoro-1,2-benzisoxazol-3-yl)piperidine (yield 54%).
Free form: ¹H NMR (CDCl₃)δ 1.73-1.84 (2H, m, CH₂), 2.01-2.08 (2H, m, CH₂), 2.21-2.30 (2H, m, CH₂), 2.33 (3H, s, CH₃), 2.67-2.77 (1H, m, piperidine CH), 2.79 (2H, t, J = 7.4 Hz, CH₂), 3.07-3.13 (2H, m, CH₂), 3.47 (2H, t, J = 7.4 Hz, CH₂), 6.06 (1H, s, = CH), 6.63 (1H, s, = CH), 6.99 (1H, dt, J = 8.7, 2.3 Hz, Ar), 7.17 (1H, dd, J = 9.0, 2.3 Hz, Ar), 7.38 (1H, s, Ar), 7.49 (1H, dd, J = 8.7, 5.4 Hz, Ar); MW 411.47 (C₂₂H₂₂N₃O₂SF); mass spectrum SIMS, m/z 412 (M+1)⁺; m.p. 125-127°C.

The title compound in a free form thus obtained was dissolved in dioxane, and a solution of 4 N HCl in dioxane was added to the solution to give a hydrochloride.
Hydrochloride: MW 447.93 (C₂₂H₂₃N₃O₂SClF); mass spectrum SIMS, m/z 411 (M+1-HCl)⁺; m.p. 210°C (dec.).

### Example 35

### 3-[2-[4-(6-Fluorobenzo[b]thiophen-3-yl)-1-piperidinyl]ethyl]-5H-7-methylthiazolo[3,2-a]pyrimidin-5-one

The title compound was synthesized in the same manner as in Example 2, except that 3-(2-chloroethyl)-5H-7-methylthiazolo[3,2-a]pyrimidin-5-one was used instead of 2-acetylamino-5-(2-chloroethyl)-4-methylthiazole and, further, 4-(6-fluorobenzo[b]thiophen-3-yl)piperidine was used instead of 4-(6-fluoro-1,2-benzisoxazol-3-yl)piperidine (yield 49%).
¹H NMR (CDCl₃)δ 1.76-1.88 (2H, m, CH₂), 2.01-2.09 (2H, m, CH₂), 2.24-2.34 (2H, m, CH₂), 2.33 (3H, d, J = 0.6 Hz, CH₃), 2.81 (2H, t, J = 7.5 Hz, CH₂), 2.84-2.94 (1H, m, piperidine CH), 3.12-3.18 (2H, m, CH₂), 3.48 (2H, t, J = 7.5 Hz, CH₂), 6.07 (1H, d, J = 0.8 Hz, = CH), 6.64 (1H, s, = CH), 7.06 (1H, d, J = 0.8 = CH), 7.13 (1H, dt, J = 8.9, 2.5 Hz, Ar), 7.53 (1H, dd, J = 8.6, 2.5 Hz, Ar), 7.69 (1H, dd, J = 8.9, 3.6 Hz, Ar); MW 427.53 (C₂₂H₂₂N₃OS₂F); mass spectrum SIMS, m/z 429 (M+2)⁺; m.p. 147-149°C.

### Example 36

### 3-[2-[4-(6-Fluoro-1,2-benzisoxazol-3-yl)-1-piperidinyl]propyl]-5H-7-methylthiazolo[3,2-a]pyrimidin-5-one

The title compound was synthesized in the same manner as in Example 2, except that 3-(3-chloropropyl)-5H-7-methylthiazolo[3,2-a]pyrimidin-5-one was used instead of 2-acetylamino-5-(2-chloroethyl)-4-methylthiazole.
¹H NMR (CDCl₃)δ 1.90-2.14 (8H, m, CH₂), 2.32 (3H, s, CH₃), 2.49 (2H, t, J = 7.2, 7.6 Hz, CH₂), 3.04-3.07 (3H, m, CH₂ and CH), 3.27 (2H, t, J = 7.2, 7.6 Hz, CH₂), 6.06 (1H, s, Ar), 6.46 (1H, s, Ar), 7.05 (1H, ddd, J = 2.3, 8.8, 8.9 Hz, Ar), 7.23 (1H, dd, J = 2.3, 8.8 Hz, Ar), 7.69 (1H, dd, J = 5.3, 8.8 Hz, Ar); MW 426 (C₂₁H₂₁N₄O₂SF); mass spectrum EIMS m/z 426 (M⁺)

### Example 37

### 3-[2-[4-(6-Fluorobenzo[b]furan-3-yl)-1-piperidinyl]ethyl]-5H-7-ethylthiazolo[3,2-a]pyrimidin-5-one

The title compound was synthesized in the same manner as in Example 2, except that 3-(2-chloroethyl)-5H-7-ethylthiazolo[3,2-a]pyrimidin-5-one was used instead of 2-acetylamino-5*-*(2-chloroethyl)-4-methylthiazole and, further, 4-(6-fluorobenzo[b]furan-3-yl)piperidine was used instead of 4-(6-fluoro-1,2-benzisoxazol-3-yl)piperidine.
¹H NMR (CDCl₃)δ 1.26 (3H, t, J = 7.6 Hz, -CH₂-CH₃), 1.79 (2H, m, J = 7.5 Hz, CH₂), 2.05 (2H, m, CH₂), 2.26 (2H, m, CH₂), 2.59 (2H, q, J = 7.6 Hz, -CH₂-CH₃), 2.72 (1H, m, piperidine CH), 2.79 (2H, t, J = 7.0 Hz, CH₂), 3.10 (2H, m, CH₂), 3.47 (2H, t, J = 7.0 Hz, CH₂), 6.07 (1H, s, Ar), 6.62 (1H, s, Ar), 6.99 (1H, ddd, J = 9.4, 8.6, 2.2 Hz, Ar), 7.17 (1H, dd, J = 9.1, 2.2 Hz, Ar), 7.38 (1H, s, Ar), 7.49 (1H, dd, J = 8.6, 5.3 Hz, Ar); MW 425.50 (C₂₃H₂₄N₃O₂SF); mass spectrum SIMS, m/z 425 (M)⁺.

### Example 38

### 3-[2-[4-(6-Fluorobenzo[b]furan-3-yl)-1-piperidinyl]ethyl]-5H-7-propylthiazolo[3,2-a]pyrimidin-5-one

The title compound was synthesized in the same manner as in Example 2, except that 3-(2-chloroethyl)-5H-7-propylthiazolo[3,2-a]pyrimidin-5-one was used instead of 2*-*acetylamino-5-(2-chloroethyl)-4-methylthiazole and, further, 4-(6-fluorobenzo[b]furan-3-yl)piperidine was used instead of 4-(6-fluoro-1,2-benzisoxazol-3-yl)piperidine
¹H NMR (CDCl₃)δ 0.98 (3H, t, J = 7.5 Hz, -CH₂-CH₃), 1.72 (2H, sextet, J = 7.5 Hz, CH₂-CH₂-CH₃), 1.79 (2H, m, CH₂), 2.04 (2H, m, CH₂), 2.26 (2H, m, CH₂), 2.52 (2H, t, J = 7.5 Hz, -CH₂-C₂H₅), 2.72 (1H, m, piperidine CH), 2.79 (2H, t, J = 7.4 Hz, CH₂), 3.10 (2H, m, CH₂), 3.47 (2H, t, J = 7.4 Hz, CH₂), 6.06 (1H, s, Ar), 6.62 (1H, s, Ar), 6.99 (1H, ddd, J = 9.2, 8.7, 2.2 Hz, Ar), 7.17 (1H, dd, J = 9.0, 2.2 Hz, Ar), 7.38 (1H, s, Ar), 7.49 (1H, dd, J = 8.7, 5.5 Hz, Ar); MW 439.53 (C₂₄H₂₆N₃O₂SF); mass spectrum SIMS, m/z 439 (M)⁺.

### Example 39

### 3-[2-[4-(6-Fluorobenzo[b]furan-3-yl)-1-piperidinyl]ethyl]-5H-7-isopropylthiazolo[3,2-a]pyrimidin-5-one

The title compound was synthesized in the same manner as in Example 2, except that 3-(2-chloroethyl)-5H-7-isopropylthiazolo[3,2-a]pyrimidin-5-one was used instead of 2-acetylamino-5-(2-chloroethyl)-4-methylthiazole and, further, 4-(6-fluorobenzo[b]furan-3-yl)piperidine was used instead of 4-(6-fluoro-1,2-benzisoxazol-3-yl)piperidine (yield 43%).
Free form: ¹H NMR (CDCl₃)δ 1.25 (6H, d, J = 6.9 Hz, CH (CH₃)₂), 1.73-1.87 (2H, m, CH₂), 2.01-2.09 (2H, m, CH₂), 2.22-2.31 (2H, m, CH₂), 2.68-2.83 (4H, m, CH, CH₂, piperidine CH), 3.07-3.14 (2H, m, CH₂), 3.47 (2H, t, J = 7.4 Hz, CH₂), 6.07 (1H, s, = CH), 6.62 (1H, s, = CH), 6.99 (1H, dt, J = 9.0, 2.3 Hz, Ar), 7.17 (1H, dd, J = 9.0, 2.3 Hz, Ar), 7.38 (1H, s, Ar), 7.50 (1H, dd, J = 9.0, 5.4 Hz, Ar); MW 439.53 (C₂₄H₂₆N₃O₂SF); mass spectrum EIMS, m/z 439 (M)⁺

The title compound in a free form thus obtained was dissolved in dioxane, and a solution of 4 N HCl in dioxane was added to the solution to give a hydrochloride.
Hydrochloride; MW 439 (C₂₄H₂₇N₃O₂SClF); mass spectrum EIMS, m/z 439 (M-HCl)⁺; m.p. 217°C (dec.).

### Example 40

### 3-[2-[4-(6-Fluoro-1,2-benzisoxazol-3-yl)-1-piperidinyl]ethyl]-5H-7-isopropylthiazolo[3,2-a]pyrimidin-5-one

The title compound was synthesized in the same manner as in Example 2, except that 3-(2-chloroethyl)-5H-7-isopropylthiazolo[3,2-a]pyrimidin-5-one was used instead of 2*-*acetylamino-5-(2-chloroethyl)-4-methylthiazole (yield 69%).
Free form: ¹H NMR (CDCl₃)δ 1.25 (6H, d, J = 6.9 Hz, CH (CH₃)₂), 2.02-2.12 (4H, m, CH₂), 2.25-2.34 (2H, m, CH₂), 2.78 (1H, q, J = 6.9 Hz, CH), 2.80 (2H, t, J = 6.9 Hz, CH₂) 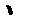 3.03-3.18 (3H, m, CH₂, piperidine CH), 3.47 (2H, t, J = 6.9 Hz, CH₂), 6.08 (1H, s, = CH), 6.63 (1H, s, = CH), 7.06 (1H, dt, J = 8.6, 2.2 Hz, Ar), 7.24 (1H, dd, J = 8.6, 2.2 Hz, Ar), 7.68 (1H, dd, J = 8.6, 5.0 Hz, Ar); MW 440.51 (C₂₃H₂₅N₄O₂SF); mass spectrum EIMS, m/z 440 (M)⁺; m.p. 98-101°C.

### Example 41

### 2,7-Dimethyl-3-[2-[4-(6-fluoro-1,2-benzisoxazol-3-yl)-1-piperidinyl]ethyl]-5H-thiazolo[3,2-a]pyrimidin-5-one

The title compound was synthesized in the same manner as in Example 2, except that 2,7-dimethyl-3-(2-chloroethyl)-5H-thiazolo[3,2-a]pyrimidin-5-one was used instead of 2-acetylamino-5-(2-chloroethyl)-4-methylthiazole (yield 64%).
¹H NMR (CDCl₃)δ 2.00-2.13 (4H, m, CH₂), 2.26-2.39 (2H, m, CH₂), 2.31 (3H, s, CH₃), 2.35 (3H, s, CH₃), 2.69-2.75 (2H, m, CH₂), 3.02-3.12 (1H, m, piperidine CH), 3.12-3.19 (2H, m, CH₂) 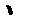 3.39-4.5 (2H, m, CH₂), 6.04 (1H, s, = CH), 7.06 (1H, dt, J = 8.7, 2.1 Hz, Ar), 7.24 (1H, dd, J = 8.5, 2.1 Hz, Ar), 7.68 (1H, dd, J = 8.7, 5.1 Hz, Ar); MW 426.49 (C₂₂H₂₃N₄O₂SF); mass spectrum EIMS, m/z 426 (M)⁺; m.p. 172-174°C

### Example 42

### 3-[2-[4-(6-Fluoro-1,2-benzisoxazol-3-yl)-1-piperazinyl]methyl]-5H-thiazolo[2,3-b]pyrimidin-5-one

The title compound was synthesized in the same manner as in Example 2, except that 3-(2-chloromethyl)-5H-thiazolo[3,2-a]pyrimidin-5-one was used instead of 2-acetylamino-5-(2-chloroethyl)-4-methylthiazole.
¹H NMR (CDCl₃)δ 2.04-2.13 (4H, m, CH₂), 3.07-3.17 (3H, m, CH₂ and CH), 4.26 (2H, d, J = 1.6 Hz, N-CH₂), 6.21 (1H, d, J = 6.6 Hz, Ar), 6.98 (1H, t, J = 1.2 Hz, Ar), 7.06 (1H, ddd, J = 2.4, 8.9, 9.0 Hz, Ar), 7.25 (1H, dd, J = 2.4, 9.0 Hz, Ar), 7.68 (1H, dd, J = 5.5, 9.0 Hz, Ar), 7.88 (1H, d, J = 6.6 Hz, Ar); MW 384.43 (C₁₉H₁₇N₄O₂SF); mass spectrum EIMS m/z 384 (M⁺).

### Example 43

### 3-[2-[4-(6-Fluoro-1,2-benzisoxazol-3-yl)-1-piperazinyl]methyl]-7H-thiazolo[2,3-b]pyrimidin-7-one

The title compound was synthesized in the same manner as in Example 2, except that 3-(2-chloromethyl)-7H-thiazolo[3,2-a]pyrimidin-7-one was used instead of 2-acetylamino-5-(2-chloroethyl)-4-methylthiazole.
¹H NMR (CDCl₃)δ 2.04-2.15 (4H, m, CH₂), 2.28-2.34 (2H, m, CH₂), 3.00-3.03 (2H, m, CH₂), 3.11-3.17 (1H, m, CH), 3.64 (2H, s, N-CH₂), 6.41 (1H, d, J = 7.8 Hz, Ar), 6.68 (1H, s, Ar), 7.07 (1H, ddd, J = 2.0, 8.6, 8.8 Hz, Ar), 7.25 (1H, dd, J = 2.0, 8.6 Hz, Ar), 7.62 (1H, dd, J = 5.1, 8.6 Hz, Ar), 8.28 (1H, d, J = 7.8 Hz, Ar); MW 384.43 (C₁₉H₁₇N₄O₂SF); mass spectrum EIMS m/z 384 (M⁺).

The structures of the above compounds are listed in the following table.

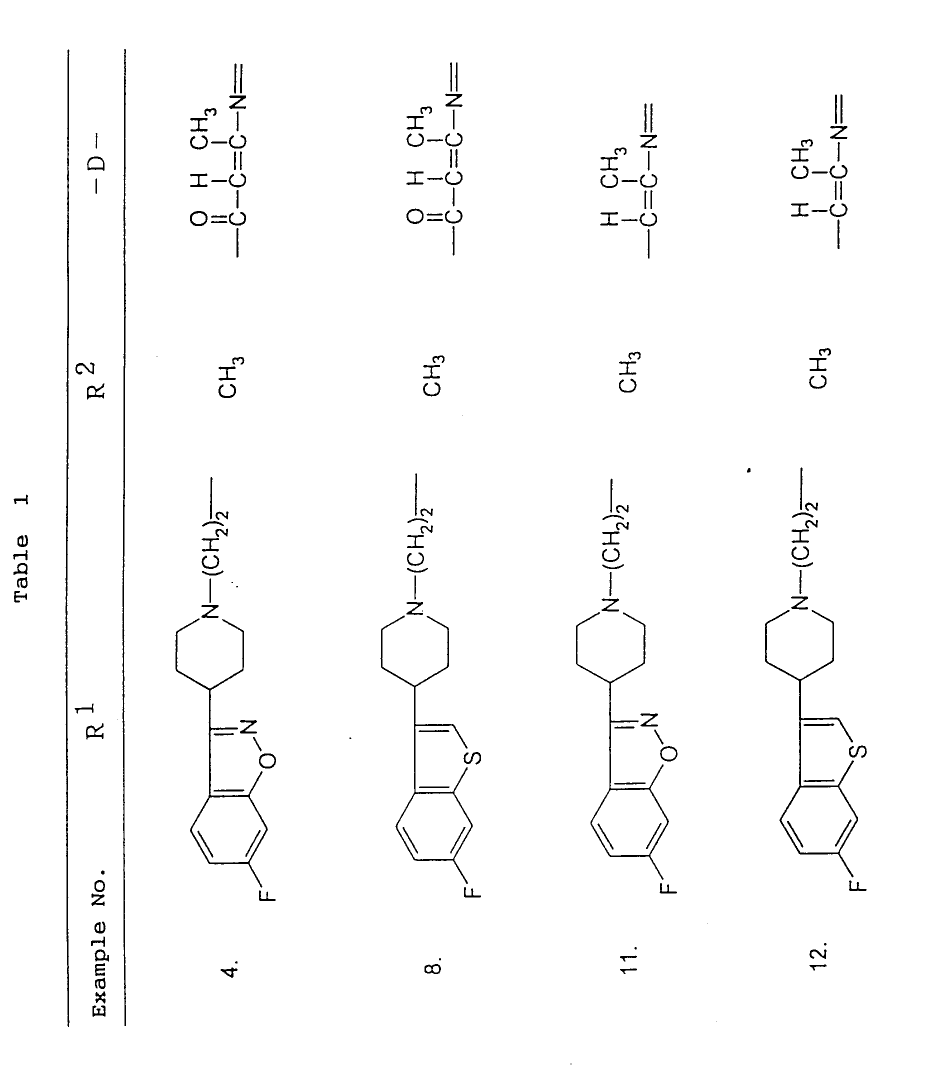

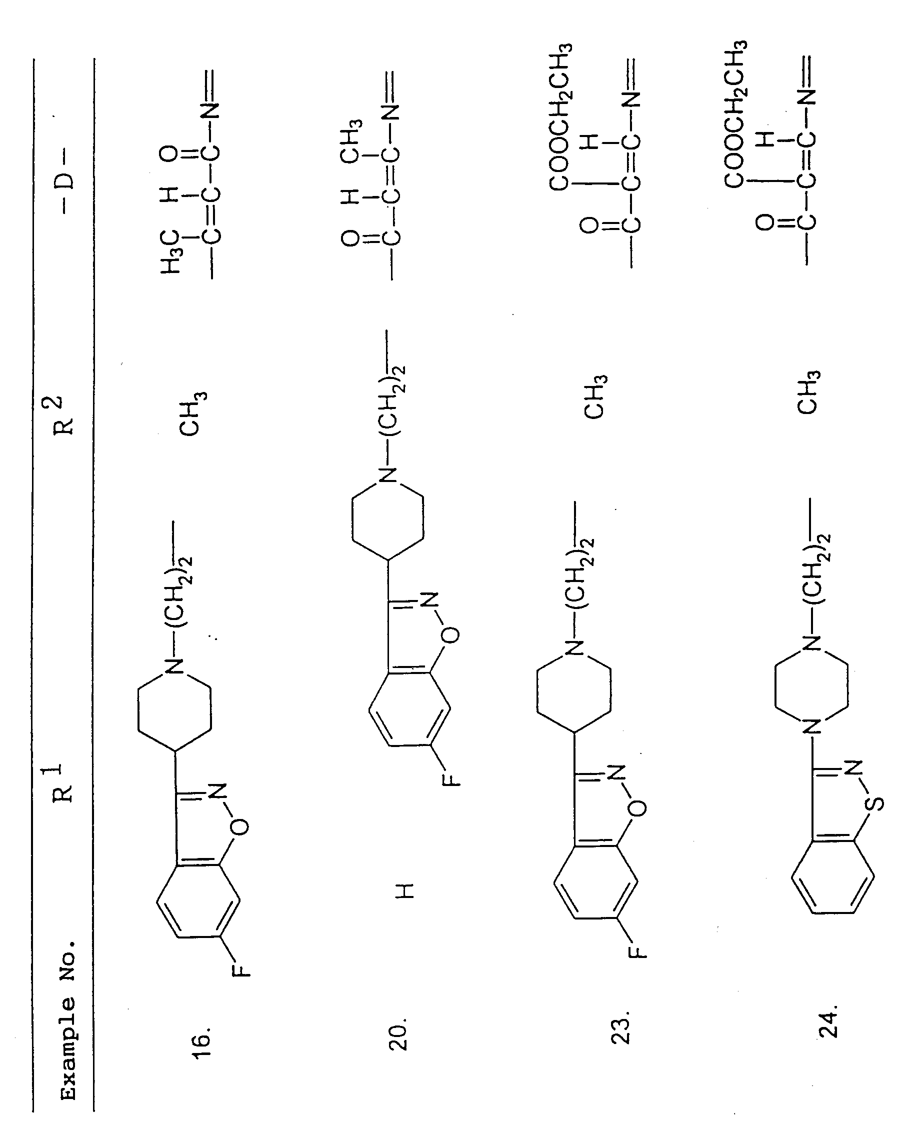

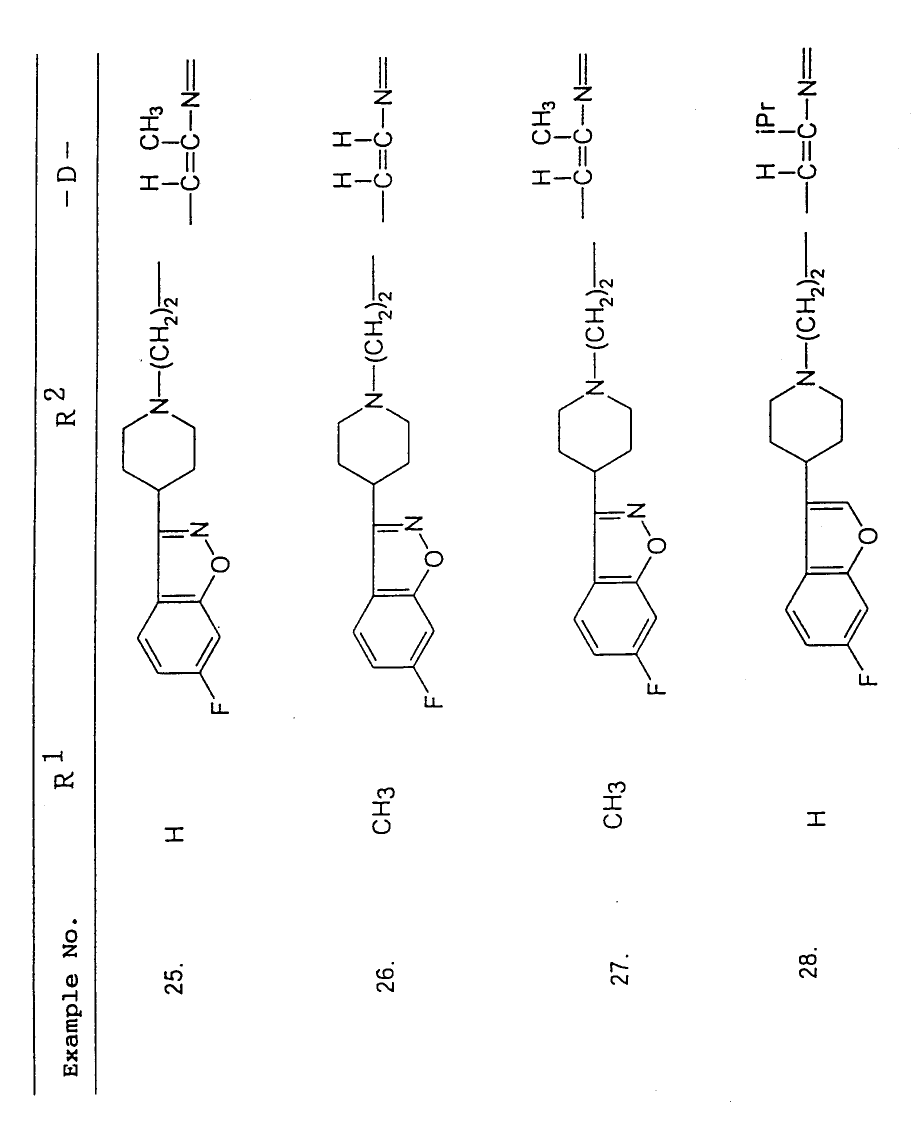

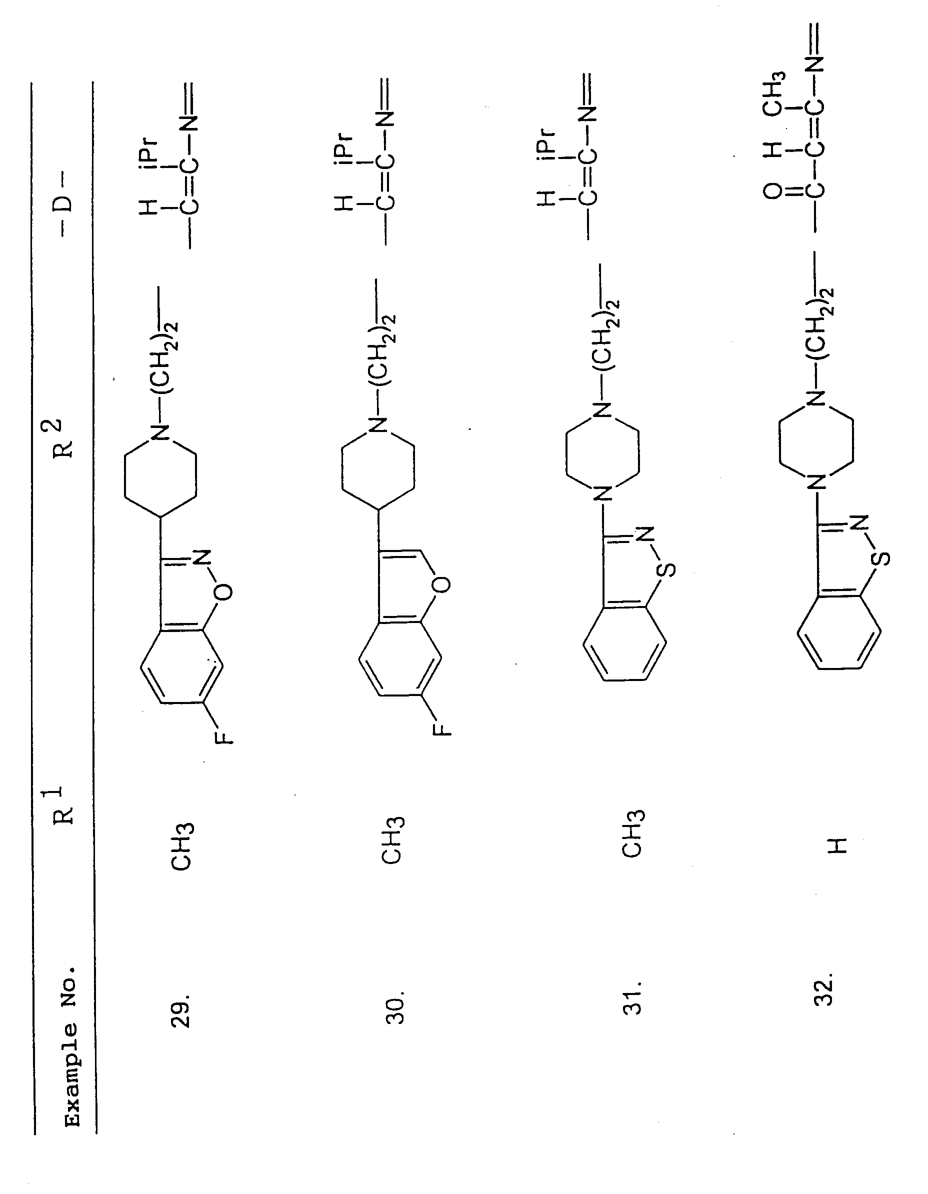

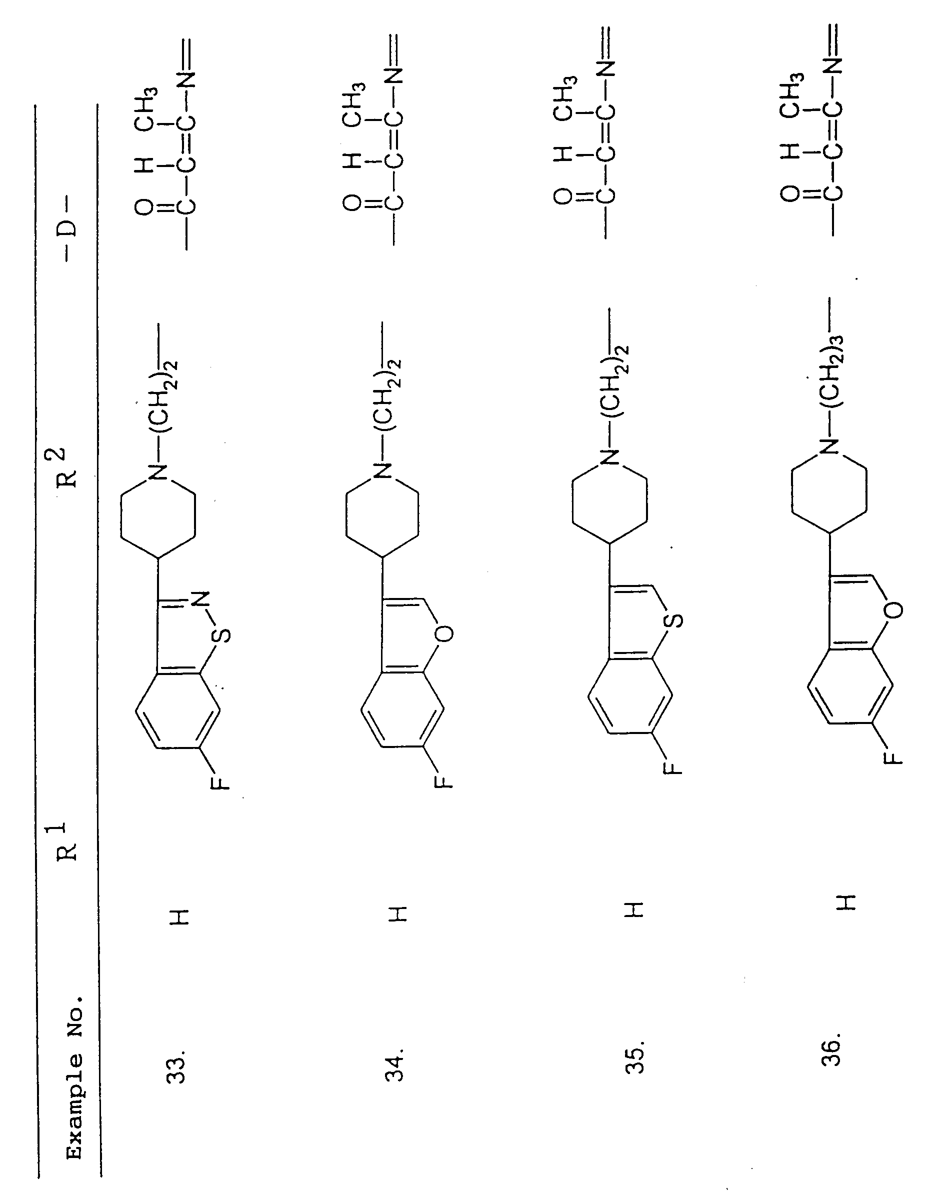

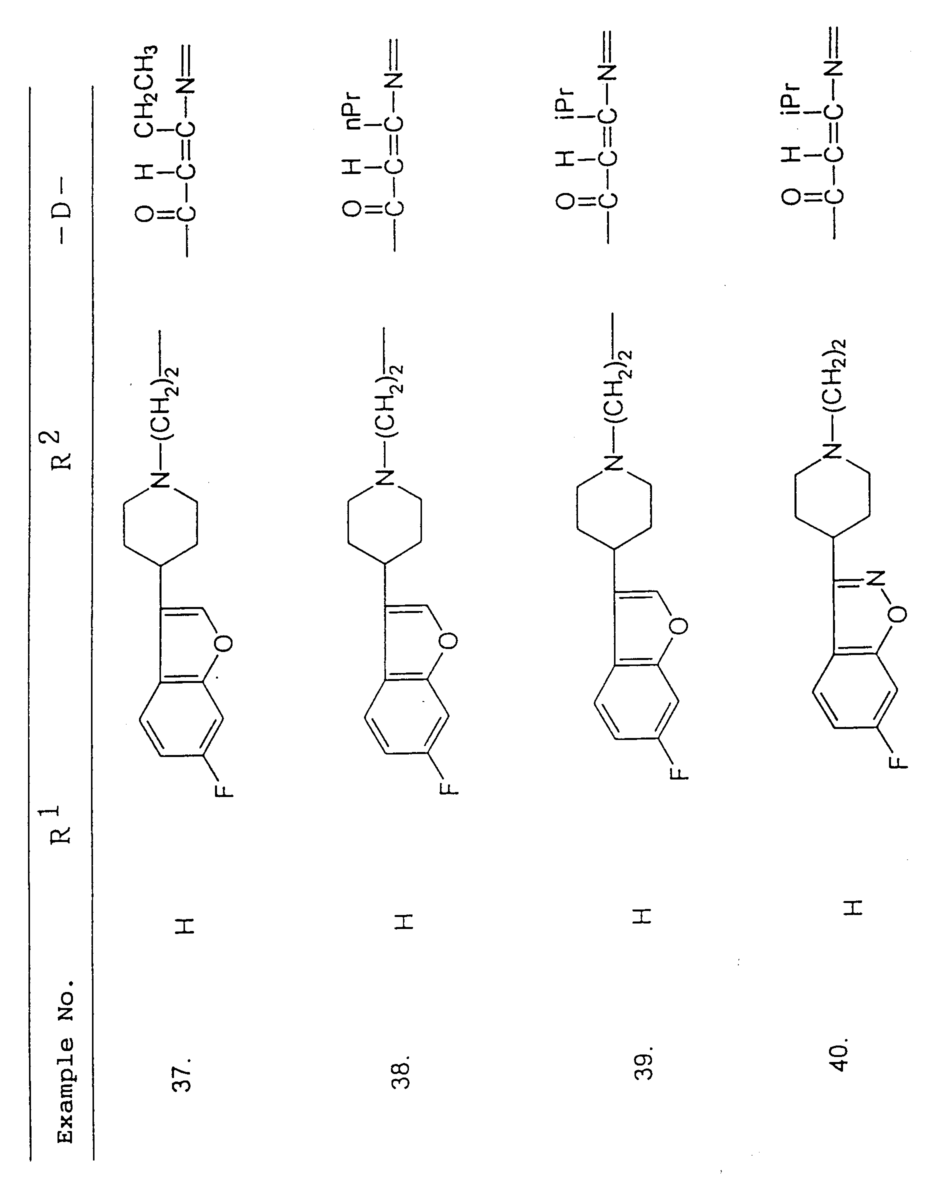

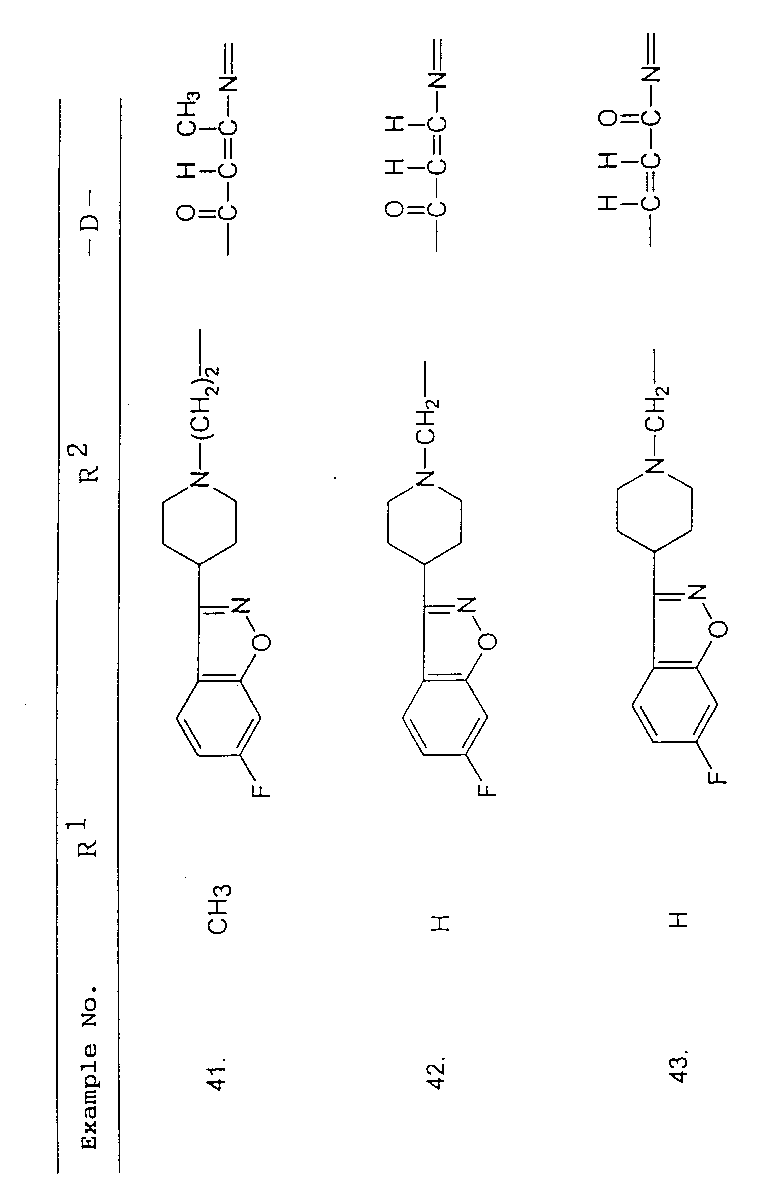

### Biological Test

### (1) Antipsychotic Activity

The antipsychotic activity of the compound according to the present invention was assayed by an index the inhibitory activity on the stimulation of hyperlocomotion induced by methamphetamine in mice. Male ddY mice having a weight of 25 - 35 g were divided into groups each consisting of 3 - 6 animals. The mouse was subcutaneously administered with methamphetamine (2 mg/kg). Fifteen minutes after this administration, the compound of the present invention was intraperitoneally administered. After further fifteen minutes, the animal was then placed in a transparent acrylic box (30 cm x 30 cm x 30 cm) mounted on an apparatus to measure the locomotion for thirty minutes (MUROMACHI KIKAI, ANIMEX AUTO MK-110). The effects of haloperidol and chlorpromazine were also evaluated as controls in the same manner as described above.

As a result, all the compounds, according to the present invention, listed in Table 1 had an ED₅₀ value of less than 30 mg/kg. Part of the results were as summarized in Table 2.

**Table 2**

| Anti-methamphetamine effect | |
|---|---|
| Compound | ED₅₀(mg/kg, ip) |
| Example 4 | 0.07 |
| Example 11 | 0.12 |
| Example 20 | 0.04 |
| Haloperidol | 0.2 |
| Chlorpromazine | 1.1 |

### (2) Extrapyramidal Effect

The extrapyramidal effect of the compounds according to the present invention was assayed by an index the catalepsy provoking activity as a representative pharmacological evaluation method of the extrapyramidal effect. Male ddY mice having a body weight of 25 - 35 g which were divided into groups each consisting of 3 - 6 animals were subjected to the test. The compound of the present invention was administered intraperitoneally to observe catalepsy after 20, 30 and 40 minutes, respectively. Catalepsy was judged as positive in such cases that a mouse whose front paw was compulsorily hung up on an iron bar having a diameter of 1 mm and horizontally laid at a height of 3 cm remained in such a strained posture for more than 30 seconds. The effects of haloperidol and chlorpromazine as the controls were also evaluated in the similar manner. The results are shown in Table 3.

**Table 3**

| Cataleptogenic Effect | |
|---|---|
| Compound | ED₅₀(mg/kg, ip) |
| Example 4 | 1.2 |
| Example 11 | 3.3 |
| Haloperidol | 1.3 |
| Chlorpromazine | 6.2 |

### (3) Binding property to various receptors

### (a) Binding property to dopamine-D2 receptors

The affinity of the compound of the present invention for D2 receptors was studied using a P2 fraction of a rat cerebral corpus striatum. The rat cerebral corpus striatum was homogenized in 0.32 M sucrose in an amount of 10 times that of the rat cerebral corpus striatum, and the homogenate was centrifuged at 900 x g for 10 min. The supernatant was further centrifuged at 11,500 x g for 20 min. An incubation buffer (50 mM Tris, 120 mM NaCl, 5 mM KCl, 1 mM MgCl₂, 1 mM CaCl₂, pH 7.4) was added to the sediment, and the mixture was then centrifuged at 39,000 x g for 20 min. The sediment was obtained as a P2 fraction. The P2 fraction was incubated at 37°C for 30 min in a buffer containing 0.1 nM [³H]spiperone and the compound of the present invention with various concentrations. The reaction solution was filtered through a Whatman GF/B glass filter, and the amount of bound [³H]spiperone was measured by a liquid scintillation counter. The amount of nonspecific binding was measured in the copresence of 10⁻⁵ M sulpiride to determine the percentage inhibition of binding of [³H]spiperone to D2 receptor by the compound of the present invention having a concentration of 10⁻⁷ M. The results are shown in Table 4.

**Table 4**

| Binding activity to dopamine-D2 receptor | |
|---|---|
| Compound | Inhibition at 10⁻⁷ M (%) |
| Example 8 | 95.2 |
| Chlorpromazine | 93.6 |

### (b) Binding activity to serotonin-5-HT₂ receptor

The affinity of the compound of the present invention for a 5-HT₂ receptor of a rat cerebral cortex was assayed as follows. A P2 fraction was prepared in the same manner as described above in connection with the above (a). The P2 fraction was incubated at 37°C for 15 min in a 50 mM Tris/HCl buffer (pH 7.4) containing 1 nM [³H]ketanserin and the compound of the present invention with various concentrations, and the amount of bound [³H]ketanserin was then measured. The amount of nonspecific binding was measured in the copresence of 10⁻⁵ M ketanserin to determine the percentage inhibition of binding of [³H]ketanserin to 5-HT₂ receptors by the compound of the present invention having a concentration of 10⁻⁹ M. The results are shown in Table 5.

**Table 5**

| Binding activity to serotonin-5-HT₂ receptor | |
|---|---|
| Compound | Inhibition at 10⁻⁹ M (%) |
| Example 20 | 66.3 |
| Chlorpromazine | 12.4 |

From the above results, it is understood that although haloperidol and chlorpromazine, which have been assayed as comparative control pharmaceuticals, have antimethamphetamine activity, that is, antipsychoic activity, they unfavorably have strong catalepsy-inducing activity, that is, extrapyramidal effect.

By contrast, it is apparent that among the compounds of the present invention, for example, the compound of Example 11 has stronger antipsychoic activity than the comparative control pharmaceutical but is poor in extrapyramidal activity. Therefore, the compounds of the present invention can be said to be usable as an antipsychotic agent having a wide safety margin.

Further, in addition of the compound having high affinity of binding, to D₂ receptors, which is considered to be an important mechanism for antipsychoic activity (for example, the compound of Example 8), compounds having high affinity for a 5-HT receptor, which is considered to be associated with central nervous-related psychoneurosis, including depression, anxiety neurosis and schizophrenia, and the like have been found (for example, the compound of Example 20) among the compounds according to the present invention. Therefore, the compounds of the present invention are considered applicable to central nervous diseases in which D₂ and 5-HT receptors are involved.

## Claims

1. A compound represented by the following formula (I) or a pharmaceutically acceptable salt thereof: 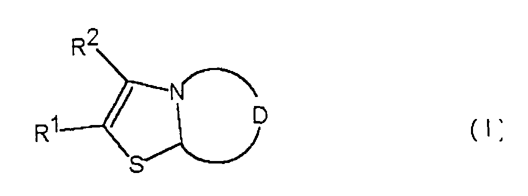 wherein
any one of R¹ and R² represents group W*-*(CH₂)ₘ- wherein m is an integer of 1 to 4 with the other representing H or optionally substituted lower alkyl,
group W is a group represented by the following formula (i): 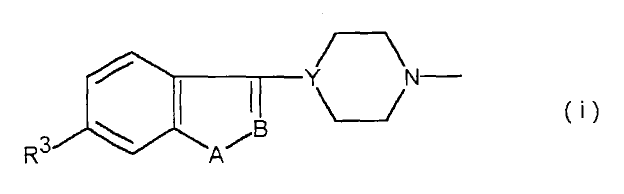 wherein
R³ represents H or a halogen atom,
A represents O or S,
B represents N or CH, and
Y represents CH or N; and
ring structure -D- represents any one of the following formulae (a) to (d): 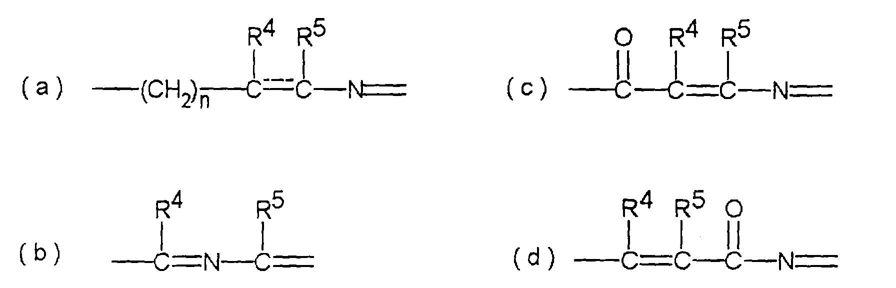 wherein
n is an integer of 0 to 2,
R⁴ and R⁵ each independently represent H, lower alkyl, a hydroxyl group, alkoxy, alkoxycarbonyl, or aminocarbonyl, and
a solid line accompanied by a dotted line represents a single bond or a double bond.

2. The compound according to claim 1, wherein, in the formula (i), A represents O, B represents N and Y represents CH.

3. The compound according to claim 1, wherein, in the formula (i), A represents S, B represents N and Y represents CH or N.

4. The compound according to claim 1, wherein, in the formula (i), A represents O or S, B represents CH and Y represents CH.

5. The compound according to claim 2 or 4, wherein the ring structure -D- represents group (a) or (b).

6. The compound according to claim 2 or 4, wherein the ring structure -D- represents group (c) or (d).

7. The compound according to any one of claims 1 to 6, wherein any one of R¹ or R² represents alkyl which may be optionally substituted by a halogen atom, a hydroxyl group, acyloxy, alkoxy, lower alkylsulfonyloxy, or aryloxysulfonyloxy.

8. The compound according to claim 1, which is selected from
3,7-dimethyl-2-[2-[4-(6-fluoro-1,2-benzisoxazol-3-yl)-1-piperidinyl]ethyl]-5H-thiazolo[3,2-a]pyrimidin-5-one,
3,7-dimethyl-2-[2-[4-(6-fluoro-1-benzothiophen-3-yl)-1-piperidinyl]ethyl]-5H-thiazolo[3,2-a]pyrimidin-5-one,
3,6-dimethyl-2-[2-[4-(6-fluoro-1,2-benzisoxazol-3-yl)-1-piperidinyl]ethyl]imidazo[2,1-b]thiazole,
3,6-dimethyl-2-[2-[4-(6-fluoro-1-benzothiophen-3-yl)-1-piperidinyl]ethyl]imidazo[2,1-b]thiazole,
3,5-dimethyl-2-[2-[4-(6-fluoro-1,2-benzisoxazol-3-yl)-1-piperidinyl]ethyl]-7H-thiazolo[3,2-a]pyrimidin-7-one,
3-[2-[4-(6-fluoro-1,2-benzisoxazol-3-yl)-1-piperidinyl]ethyl]-5H-7-methylthiazolo[3,2-a]pyrimidin-5-one,
6-ethoxycarbonyl-2-[2-[4-(6-fluoro-1,2-benzisoxazol-3-yl)-1-piperidinyl]ethyl]-5H-3-methylthiazolo[3,2-a]pyrimidin-5-one,
6-ethoxycarbonyl-2-[2-[4-(1,2-benzoisothiazol-3-yl)-1-piperazinyl]ethyl]-5H-3-methylthiazolo[3,2-a]pyrimidin-5-one,
3-[2-[4-(6-fluoro-1,2-benzisoxazol-3-yl)-1-piperidinyl]ethyl]-6-methylimidazo[2,1-b]thiazole,
3-[2-[4-(6-fluoro-1,2-benzisoxazol-3-yl)-1-piperidinyl]ethyl]-2-methylimidazo[2,1-b]thiazole,
3-[2-[4-(6-fluoro-1,2-benzisoxazol-3-yl)-1-piperidinyl]ethyl]-2,6-dimethylimidazo[2,1-b]thiazole,
3-[2-[4-(6-fluorobenzo[b]furan-3-yl)-1-piperidinyl]ethyl]-6-isopropylimidazo[2,1-b]thiazole,
3-[2-[4-(6-fluoro-1,2-benzisoxazol-3-yl)-1-piperidinyl]ethyl]-6-isopropyl-2-methylimidazo[2,1-b]thiazole,
3-[2-[4-(6-fluorobenzo[b]furan-3-yl)-1-piperidinyl]ethyl]-6-isopropyl-2-methylimidazo[2,1-b]thiazole,
3-[2-[4-(1,2-benzoisothiazol-3-yl)-1-piperazinyl]ethyl]-6-isopropyl-2-methylimidazo[2,1-b]thiazole,
3-[2-[4-(1,2-benzoisothiazol-3-yl)-1-piperazinyl]ethyl]-5H-7-methylthiazolo[3,2-a]pyrimidin-5-one,
3-[2-[4-(6-fluoro-1,2-benzoisothiazol-3-yl)-1-piperidinyl]ethyl]-5H-7-methylthiazolo[3,2-a]pyrimidin-5-one,
3-[2-[4-(6-fluorobenzo[b]furan-3-yl)-1-piperidinyl]ethyl]-5H-7-methylthiazolo[3,2-a]pyrimidin-5-one,
3-[2-[4-(6-fluorobenzo[b]thiophen-3-yl)-1-piperidinyl]ethyl]-5H-7-methylthiazolo[3,2-a]pyrimidin-5-one,
3-[2-[4-(6-fluoro-1,2-benzisoxazol-3-yl)-1-piperidinyl]propyl]-5H-7-methylthiazolo[3,2-a]pyrimidin-5-one,
3-[2-[4-(6-fluorobenzo[b]furan-3-yl)-1-piperidinyl]ethyl]-5H-7-ethylthiazolo[3,2-a]pyrimidin-5-one,
3-[2-[4-(6-fluorobenzo[b]furan-3-yl)-1-piperidinyl]ethyl]-5H-7-propylthiazolo[3,2-a]pyrimidin-5-one,
3-[2-[4-(6-fluorobenzo[b]furan-3-yl)-1-piperidinyl]ethyl]-5H-7-isopropylthiazolo[3,2-a]pyrimidin-5-one,
3-[2-[4-(6-fluoro-1,2-benzisoxazol-3-yl)-1-piperidinyl]ethyl]-5H-7-isopropylthiazolo[3,2-a]pyrimidin-5-one,
2,7-dimethyl-3-[2-[4-(6-fluoro-1,2-benzisoxazol-3-yl)-1-piperidinyl]ethyl]-5H-thiazolo[3,2-a]pyrimidin-5-one,
3-[2-[4-(6-fluoro-1,2-benzisoxazol-3-yl)-1-piperazinyl]methyl]-5H-thiazolo[2,3-b]pyrimidin-5-one, and
3-[2-[4-(6-fluoro-1,2-benzisoxazol-3-yl)-1-piperazinyl]methyl]-7H-thiazolo[2,3-b]pyrimidin-7-one.

9. A pharmaceutical composition comprising as an active ingredient the compound according to any one of claims 1 to 8 or a pharmaceutically acceptable salt thereof.

10. The pharmaceutical composition according to claim 9, which is a psychotropic agent.

11. Use of the compound according to any one of claims 1 to 8 or a pharmaceutically acceptable salt thereof as a psychotropic agent.

12. Use of the compound according to any one of claims 1 to 8 or a pharmaceutically acceptable salt thereof for preparing a psychotropic agent.

13. A method for treatment of a psychosis, comprising the step of administering to mammals, including human being, the compound according to any one of claims 1 to 8 or a pharmaceutically acceptable salt thereof.

14. The method according to claim 13, wherein the psychosis is neurosis, schizophrenia, or depression.
